# EUROPEAN PATENT APPLICATION

(11) **EP 4 674 871 A1**
(43) Date of publication of application: **07.01.2026**
(21) Application number: 24764000.6
(22) Date of filing: 28.02.2024
(51) Int. Cl.: C07K 19/00, A61K 6/15, C07K 14/47, C07K 14/475, C12N 1/15, C12N 1/19, C12N 1/21, C12N 5/10, C12N 15/12, C12N 15/62, C12N 15/63, C12P 21/02

(54) **RECOMBINANT PROTEIN HAVING ADHESIVENESS AND FUNCTIONALITY, AND COMPOSITION COMPRISING SAME**

(30) Priority: 28.02.2023 JP 2023030567
(71) Applicant: Novelgen Co., Ltd., Nagahama-shi, Shiga 526-0829 (JP)
(72) Inventor: SHISHIOH, Nobue, Nagahama-shi, Shiga 526-0829 (JP); OGURA, Atsushi, Nagahama-shi, Shiga 526-0829 (JP)
(74) Representative: Calysta NV
(86) International application number: PCT/JP2024/007452
(87) International publication number: WO 2024/181523

(57) **Abstract**

Provided is an adhesive protein that has high adhesive strength and low toxicity and can be efficiently produced. In addition, there is provided an adhesive protein having adhesiveness and additional functionality useful for medical and/or research applications. A recombinant protein includes a functional peptide portion having growth factor activity; and an adhesive peptide portion fused to an N-terminal side and an adhesive peptide portion fused to a C-terminal side of the functional peptide portion.

## Description

### Technical Field

The present invention relates to a recombinant protein having adhesiveness and functionality, a method for producing the same, a composition containing the same, and the like.

### Background Art

Biocompatible materials having water resistance and adhesiveness are required in various fields such as various research and development and medical sites. In the medical field, since the inside of a living body is in a wet environment, a substance having strong adhesiveness in a wet state and exhibiting strong bioactivity is required. In particular, in the dental field, there is a case where suturing is not possible, and it is necessary to maintain adhesiveness for a long period of time in a wet environment such as an oral cavity.

Therefore, there is a demand for a substance having strong adhesive strength even in a wet state and having activity related to tissue regeneration such as a cell proliferation activity that enables early treatment/repair/regeneration of tissue at a surgical site. In addition, in the field of research and development, since a polystyrene disposable product for cell culture has a hydrophobic surface and poor affinity for cells, it is necessary to improve the adhesiveness of cells such as nerve cells, periodontal tissue, fertilized eggs and blastocysts, and hematopoietic stem cells to a surface of a culture vessel.

Among medical adhesives currently in use, a cyanoacrylate-based adhesive has high adhesive strength but has a problem of high toxicity to a living body, while a fibrin-based adhesive has no toxicity but has low adhesive strength, and cases of viral infection have been reported.

Mussels (Mytilus edulis, M. galloprovincialis, M. coruscus, and the like) secrete specific adhesive proteins, which are types of fibrous protein and have adhesive ability in water, called mussel adhesive proteins (MAPs). MAPs are also expected to be useful for medical applications due to their properties such as adhesiveness in a wet environment. In addition, for the purpose of adhering cells and tissue cross-sections to various types of surfaces such as plastics, glass, metals, fluororesins, and biological materials, polyphenol proteins secreted by The Mediterranean mussel (M. galloprovincialis) are commercially available for research purposes.

It is said that extraction of MAPs from natural mussels requires 10,000 mussels to obtain 1 g of MAPs (Non Patent Literature 1), and because this is extremely inefficient, functional recombinant MAPs have been produced using several expression systems.

For example, FP151 is a fusion protein of FP1 and FP5 derived from Mytilus galloprovincialis. A sequence characteristic is that a sequence in which a motif derived from an adhesive protein FP1 is repeated six times is added to both ends of an FP5 motif, which is an adhesive protein (Non Patent Literature 2).

### Citation List

### Patent Literature

Patent Literature 1: JP 2008-504016 A
Patent Literature 2: WO 2008/150101 A
Patent Literature 3: JP 2015-528493 A
Patent Literature 4: JP 8772617 B2
Patent Literature 5: Re-publication of WO 2019/087650 A
Patent Literature 6: JP 2020-511942 A

### Non Patent Literature

Non Patent Literature 1: Masanobu Naito, "Functional Materials Mimicking Marine Adhesive Organisms", J. Jpn. Soc. Colour Mater., 87 (1), 13-18 (2014)
Non Patent Literature 2: Hwang, et al., "Practical recombinant hybrid mussel bioadhesive fp-151", Biomaterials, vol. 28, No. 24, pp. 3560-3568, 2007

### Summary of Invention

### Technical Problem

Accordingly, there is a need for medical adhesives and coating agents that are safe and have high adhesive strength. In addition, there is no report that productivity improvement or the like has been studied for existing adhesive proteins. Therefore, they were very expensive and their practicality was limited.

An object of the present invention is to provide an adhesive protein that has high adhesive strength and low toxicity and can be efficiently produced. In addition, an object of the present invention is to provide an adhesive protein having adhesiveness and additional functionality useful for medical and/or research applications.

### Solution to Problem

As a result of intensive studies on the above problems, the present inventors have completed the present invention.

That is, according to the present invention, there are provided
[1] A recombinant protein including: a functional peptide portion having growth factor activity; and an adhesive peptide portion fused to an N-terminal side and an adhesive peptide portion fused to a C-terminal side of the functional peptide portion;
[2] The recombinant protein according to [1], in which the functional peptide portion is a functional peptide derived from a human;
[3] The recombinant protein according to [1], in which the adhesive peptide portion on the N-terminal side or the C-terminal side is an adhesive peptide derived from a mussel;
[4] The recombinant protein according to [1], in which two or more of the adhesive peptide portions are present on each of the N-terminal side and the C-terminal side of the functional peptide portion;
[5] The recombinant protein according to [1], in which the functional peptide portion has cell proliferation-stimulating activity;
[6] The recombinant protein according to [1], in which the functional peptide portion has an amino acid sequence set forth in any one of SEQ ID NOs: 1 to 12, 79, or 80;
[7] The recombinant protein according to [1], in which the adhesive peptide portion has an amino acid sequence set forth in any one of SEQ ID NOs: 13 to 23;
[8] A nucleic acid encoding the recombinant protein according to any one of [1] to [7];
[9] An expression vector containing the nucleic acid according to [8] in a configuration capable of expression;
[10] A transformant transformed with the expression vector according to [9];
[11] A method for producing the recombinant protein, the method including culturing a transformant comprising the expression vector according to [9] and recovering the recombinant protein from the culture;
[12] A composition containing the recombinant protein according to any one of [1] to [7];
[13] The composition according to [12], in which the composition is a medical adhesive;
[14] The composition according to [12], in which the composition is a coating agent for cell culture; and
[15] The composition according to [12], in which the composition is a cell proliferation-stimulating agent.

### Advantageous Effects of Invention

According to the present invention, it is possible to efficiently prepare a low-toxicity and safe recombinant protein having functionality and high adhesiveness that can be used for medical and/or research applications and the like by a simple process.

### Brief Description of Drawings

[Fig. 1] Fig. 1 is a schematic diagram illustrating the structures of the constructs of respective expression vectors (Examples 1 to 8), which is one embodiment of the present invention.
[Fig. 2] Fig. 2 is a diagram illustrating the preparation scheme of the insert fragment containing FP121-9 gene.
[Fig. 3] Fig. 3 is a photograph showing the SDS-PAGE of the purified recombinant protein FP121-6 (prototype) (see Example 10 for details).
[Fig. 4] Fig. 4 is a photograph showing an SDS-PAGE of the expressed recombinant proteins FP121-1, FP121-3, FP121-6, and FP121-9 (see Example 11 for details).
[Fig. 5] Fig. 5 is a diagram illustrating the protein yield per 1 g of cells and the proportion of recombinant protein for various transformants. The solid bars indicate the mass of extracted protein, the hatched bars indicate the mass of purified recombinant protein, and the triangles indicate the proportion (%) of recombinant protein (see Example 11-3 for details).
[Fig. 6] Fig. 6 is a photograph showing the SDS-PAGE of the purified recombinant protein FP121-3 (see Example 11-3 for details).
[Fig. 7] Fig. 7 is a photograph showing the SDS-PAGE of the purified recombinant protein FP121-6 (see Example 11-3 for details).
[Fig. 8] Fig. 8 is a photograph showing the SDS-PAGE of the purified recombinant protein FP121-9 (see Example 11-3 for details).
[Fig. 9] Fig. 9 is a photograph showing the SDS-PAGE of the expressed recombinant proteins FP120 and FP021 (see Example 12 for details).
[Fig. 10] Fig. 10 is a diagram illustrating the protein yield per 1 g of cells and the proportion of recombinant protein for various transformants. The solid bars indicate the mass of extracted protein, the hatched bars indicate the mass of purified recombinant protein, and the triangles indicate the proportion (%) of recombinant protein (see Example 12-2 for details).
[Fig. 11] Fig. 11 is a photograph showing the SDS-PAGE of the purified recombinant protein FP120 (see Example 12-3 for details).
[Fig. 12] Fig. 12 is a photograph showing the SDS-PAGE of the purified recombinant protein FP021 (see Example 12-3 for details).
[Fig. 13] Fig. 13 is a photograph showing the SDS-PAGE of the expressed recombinant proteins hEGF-FP1-6 and cbFP121 (see Example 13-2 for details).
[Fig. 14] Fig. 14 is a photograph showing the SDS-PAGE of the purified recombinant protein cbFP121-6 (see Example 13-4 for details).
[Fig. 15] Fig. 15 is a photograph showing the SDS-PAGE of the purified recombinant protein hEGF-FP1-6 (see Example 13-5 for details).
[Fig. 16] Fig. 16 is a photograph showing the SDS-PAGE (NBT-stained image) for confirming the DOPA modification of the recombinant protein FP121-6 (prototype) (see Example 14-3 for details).
[Fig. 17] Fig. 17 is a diagram illustrating the effects of various recombinant proteins on the cell adhesion rate of suspension K562 cell line (see Example 15 for details). In the drawing, "FP121-6 (proto)" represents FP121-6 (prototype) (the same applies hereinafter).
[Fig. 18] Fig. 18 is a diagram illustrating the cell proliferation-stimulating effect on HeLa cells by various recombinant proteins (see Example 16 for details).
[Fig. 19] Fig. 19 is a diagram illustrating the effects of various recombinant proteins on the cell adhesion rate of suspension K562 cell line (see Example 17 for details).
[Fig. 20] Fig. 20 is a diagram illustrating the cell proliferation-stimulating effect on HeLa cells by various recombinant proteins (see Example 18 for details).
[Fig. 21] Fig. 21 is a diagram illustrating the effects of various recombinant proteins on the cell adhesion rate of suspension K562 cell line (see Example 19 for details).
[Fig. 22] Fig. 22 is a diagram illustrating the cell proliferation-stimulating effect on HeLa cells by various recombinant proteins (see Example 20 for details).
[Fig. 23] Fig. 23 is a diagram illustrating the effects of the recombinant protein hEGF-FP1-6 on the cell adhesion rate of suspension K562 cell line (see Example 21 for details).
[Fig. 24] Fig. 24 is a diagram illustrating the cell proliferation-stimulating effect on HeLa cells by the recombinant protein hEGF-FP1-6 (see Example 22 for details).
[Fig. 25] Fig. 25 is a diagram illustrating the adhesion rate of suspension K562 cells after long-term storage.
[Fig. 26] Fig. 26 is a diagram illustrating the cell proliferation effect after coating and after long-term storage.
[Fig. 27] Fig. 27 is a diagram illustrating the cell adhesion rate by acetic acid coating.
[Fig. 28] Fig. 28 is the cell culture result showing the neural differentiation-inducing ability of hNGF-FP1-6.

### Description of Embodiments

Hereinafter, the present invention will be described in detail.

### <Recombinant Protein>

A recombinant protein according to one embodiment of the present invention includes a functional peptide portion; and an adhesive peptide portion fused to an N-terminal side and an adhesive peptide portion fused to a C-terminal side of the functional peptide portion.

The functional peptide portion may have any functionality other than adhesiveness, and preferably has a function such as stimulating, inducing, promoting, or activating cell proliferation, cell growth, cell differentiation, or tissue regeneration, and more preferably has growth factor activity. In the present specification, the growth factor activity refers to an activity that promotes cell proliferation or differentiation in vivo. Examples of the functional peptide portion having growth factor activity include a growth factor, a cell stimulating factor, a regeneration factor, and peptide fragments derived from these molecules and having the functions of these molecules.

Here, the growth factor, which is also referred to as a growth factor or a cell proliferation factor, refers to an endogenous protein that promotes proliferation or differentiation of specific cells in vivo, and includes a growth factor, a hormone, and the like. Specific examples thereof include EGF, FGF (particularly FGF2 or bFGF), IGF (particularly IGF1), BMP, NGF, PDGF, VEGF, and HGF, but are not particularly limited thereto. In addition, FP2 is also included in the growth factor. FP2 is a protein known as an adhesive protein derived from Mytilus galloprovincialis, and in the present invention, the present inventors have found for the first time that it has a cell proliferation function.

The growth factor in the present invention includes not only the above-described proteins but also variants having similar functions.

The cell stimulating factor refers to a factor that provides stimulation such as proliferation, differentiation, survival, or migration to tissue-specific cells such as hematopoietic stem cells and hematopoietic progenitor cells. Such a cell stimulating factor is not particularly limited as long as it does not inhibit the proliferation of hematopoietic stem cells or hematopoietic progenitor cells, and specific examples thereof include stem cell factor (SCF), interleukin-3 (IL-3), granulocyte/macrophage colony-stimulating factor (GM-CSF), interleukin-6 (IL-6), soluble IL-6 receptor (sIL-6R), interleukin-11 (IL-11), fms-like tyrosine kinase 3 (Flt-3) ligand (Flt-3L), erythropoietin (EPO), thrombopoietin (TPO), granulocyte colony-stimulating factor (G-CSF), transforming growth factor-β (TGF-β), MIP-1α (George, D., J. Exp. Med. 167:1939-1944, 1988), Flt3/Flk2 ligand, and fibroblast growth factor (FGF), but are not limited thereto.

Specific examples of the regeneration factor include hepatocyte growth factor (HGF), vascular endothelial growth factor (VEGF), epidermal growth factor (EGF), insulin-like growth factor (IGF-1), stromal cell-derived factor (SDF-1), various fibroblast growth factors (a/b-FGF (also described as a/bFGF)), transforming growth factor-β (TGF-β), platelet-derived growth factor (PDGF), angiopoietin, hypoxia inducible factor (HIF-1), bone morphogenetic protein (BMP), connective tissue growth factor (CTGF), nerve growth factor (NGF), brain-derived neurotrophic factor (BDNF), glial cell-derived neurotrophic factor (GDNF), stem cell factor (SCF), granulocyte colony-stimulating factor (G-CSF), keratinocyte growth factor (KGF), chondrocyte growth factor (GDF), leukemia inhibitory factor (LIF), HMBG1, and Kruppel-like transcription factor (KLF), or growth factors of their families, but are not particularly limited.

The functional peptide may be any peptide as long as it can be fused with the adhesive peptide portion described below and can exert its function in vivo. The function of the functional peptide portion may be any function such as stimulating, inducing, promoting, or activating cell proliferation, cell growth, cell differentiation, or tissue regeneration, and preferably, examples thereof include functions such as stimulation of cell proliferation, stimulation of cell growth, promotion of angiogenesis, induction of differentiation into biological tissues, survival or maintenance of nerve cells, promotion of neurite elongation, angiogenesis, vasculogenesis, induction of tissue regeneration, regulation of expression of extracellular matrix, chemotactic response of cells, and regeneration, repair, formation, or differentiation induction of cartilage tissue.

The functional peptide portion may be derived from any organism, and examples thereof include mammals such as humans, dogs, cats, mice, and rats, birds such as fowl, and marine organisms such as mussels and squid. Specific examples of an amino acid sequence of a peptide that can be used as a functional peptide portion include the sequences set forth in SEQ ID NOs: 1 to 12, 79, and 80.

Note that, in the present specification, the term "peptide" refers to a polymer of amino acids and is used as a term including a dipeptide, an oligopeptide, a polypeptide, and the like, and unless otherwise specified, is not limited in length.

The term "fusion" as used in the present specification refers to the linking of different peptides or proteins by genetic or chemical methods. In this case, the bound ends of the peptides or proteins may be directly adjacent to or linked to each other, or may be adjacent to or linked to each other via a linker or spacer portion such as an amino acid residue or another linking group.

Examples of a linker sequence include a sequence containing an amino acid. In particular, examples thereof include sequences containing at least one amino acid selected from threonine, serine, proline, glycine, aspartic acid, lysine, glutamine, asparagine, alanine, and leucine. The length of the linker sequence is not particularly limited, but is preferably about 0 to 50 amino acid residues, more preferably about 4 to 30 amino acid residues, and still more preferably about 5 to 20 amino acid residues. Specific examples include linkers containing (Gly-Gly-Gly-Ser)n or (Gly-Gly-Gly-Gly-Ser)n, which are known as flexible linkers, wherein n is 1 to 10. More specifically, it is (Gly₄Ser)₄ or (Gly₄Ser)₃. Another specific example is a linker containing (Glu-Ala-Ala-Ala-Lys)n, which is known as a rigid linker, where n is 1 to 6.

In the recombinant protein according to one embodiment of the present invention, the adhesive peptide portion is present on an N-terminal side and a C-terminal side of the functional peptide portion. The adhesive peptide portion is not particularly limited as long as it has adhesiveness, but is preferably derived from an adhesive protein having an adhesive ability in water, such as MAPs of mussels, for example, an adhesive protein having a natural sequence of FP (foot protein) 1 to FP6 isolated from mussels or a variant sequence thereof, more preferably derived from an adhesive protein having a natural sequence of FP1 or FP3 to FP6 or a variant sequence thereof, and most preferably derived from an adhesive protein having a natural sequence of FP1 or a variant sequence thereof.

As the adhesive protein or adhesive peptide portion, those having an amino acid sequence containing many tyrosine residues, and those having an amino acid sequence containing hydrophobic amino acids other than tyrosine and basic amino acids are preferred, and in particular, those having an amino acid sequence containing many tyrosine residues are preferred. For example, those having an amino acid sequence containing 2% or more, 10% or more, or 20% or more tyrosine residues in the amino acid sequence of the adhesive protein or adhesive peptide portion are preferred. The content of tyrosine residues can be 40% or less, 35% or less, or 30% or less. Note that the content of tyrosine residues in the amino acid sequence is calculated on the basis that the total number of amino acid residues, excluding additional sequences such as a His-tag and a start codon, is 100%.

In addition, those having an amino acid sequence containing polylysine containing a basic amino acid or polytyrosine containing a hydrophobic amino acid can also be used as the adhesive protein or adhesive peptide portion of the present invention. Furthermore, adhesive peptides derived from adhesive proteins of barnacles, starfish, tube worms, squid, octopuses, silkworms, and the like can also be used in the same manner.

Specific examples of an amino acid sequence of a peptide that can be used as an adhesive peptide portion include the sequences set forth in SEQ ID NOs: 13 to 23.

The adhesive peptide portion may be any peptide portion as long as it can be fused with the functional peptide portion and can exert its function in vivo.

The adhesive peptide portion can be present as a repeated motif sequence on each of the N-terminal side and the C-terminal side of the functional peptide portion. Preferably, an adhesive peptide portion composed of two or more repeating motifs is present on each of the N-terminal side and the C-terminal side. The number of repeats of the adhesive peptide portion on the N-terminal side and the C-terminal side is each at least 2 or more, preferably 3 or more, and 12 or less, preferably 9 or less, more preferably 3 to 9, and most preferably 3 to 6 or 5 to 7.

The adhesive peptide portions present on the N-terminal side and the C-terminal side may be the same sequence or different sequences. In addition, the adhesive peptide portions present on the N-terminal side and the C-terminal side may have the same or different number of repeats or lengths.

The recombinant protein of one embodiment of the present invention may further have another additional sequence in addition to the essential functional peptide and adhesive peptide. Such additional sequences are not particularly limited as long as they do not impair the intended function and adhesiveness of the recombinant protein of the embodiment, and examples thereof include a histidine tag (His tag; a tag of about 6 to 10 polyhistidines) for immobilized metal affinity chromatography purification, and a calcium-binding domain (C-X-N-XXXX-Y-X-C; for example, SEQ ID NO: 24).

### <Expression Vector and Transformant>

In order to produce the recombinant protein of one embodiment of the present invention, a gene encoding a fusion protein having the amino acid sequence as described above can be synthesized by a known method.

An expression vector containing such a gene can be constructed by a known method by inserting a nucleic acid sequence containing the selected gene into a known expression vector. An expression vector having a lac promoter or a tac promoter for inducing expression is preferred. For mass expression of recombinant proteins, plasmids available in the pET system, that is, expression vectors whose expression is controlled by a T7 promoter, are particularly preferred.

The constructed expression vector can be used to transform the host to produce the recombinant protein of the embodiment.

As the host, various strains of E. coli can be used. A strain in which λ phage DE3 is lysogenized in the chromosome and which expresses T7 RNA polymerase under the control of the lacUV5 promoter is desired. Specific examples thereof include BL21(DE3), NovaBlue(DE3), and Tuner(DE3).

The medium may be any medium capable of culturing E. coli, and can be appropriately selected and used according to the host to be used. For example, a known medium such as LB medium or 2×YT medium can be used.

As a method of transforming a host with an expression vector to obtain a transformant and a method of selecting and culturing the transformant, any known method may be used. For example, an appropriate amount (for example, 5 to 50 ng) of plasmid can be introduced into E. coli by an electroporation method. The transformant can be easily selected by using an appropriate antibiotic or the like, and for example, when an adhesive protein gene is introduced into a pET22b vector, the transformant can be selected using LB medium containing ampicillin.

The culture for protein expression can be performed by scaling up and culturing the obtained transformant or a stock thereof by a known method. The culture conditions can be appropriately selected according to the host, and E coli can generally be cultured aerobically at 30 to 37°C.

### <Preparation of Recombinant Protein>

In the method of one embodiment according to the present invention, expression is induced at a specific point during culturing of the transformant, for example, when the cells have reached a high density. For example, in the case of E coli, induction can be started when the density, represented by an absorbance (OD600) at a wavelength of 600 nm, has reached 0.6 or more, for example, 0.6 to 2.0, preferably 1.0 to 2.0, and more preferably 1.5 to 2.0. When the cell density at the start of induction is high, the yield of the target protein can be increased by about 3- to 5-fold compared to when the density is low (that is, OD600 < 0.6).

Induction is started by adding isopropyl-β-D-thiogalactopyranoside (IPTG) to the medium. The final concentration of IPTG in the medium can be added to 0.1 to 1 mM. The concentration is preferably 0.1 to 0.8 mM, and more preferably 0.1 to 0.5 mM. The use of a low concentration up to about 0.1 mM is preferred because it can yield an amount of protein equal to or greater than that obtained by induction at a higher concentration, while also reducing costs.

After addition of IPTG, expression is induced by continuing the culture at the culture temperature. The induction time can be 1 to 20 hours, preferably 4 to 14 hours, more preferably 6 to 14 hours. Under certain conditions, the induction time is preferably 8 to 12 hours because the protein yield reaches its maximum at an induction time of about 10 hours.

After completion of induction, the cells are collected by a conventional method. If necessary, the collected cell pellet may be washed with a buffer or the like.

In the method according to one embodiment of the present invention, the collected cells are solubilized using acetic acid or a protein denaturant. As the protein denaturant, a chaotropic agent is preferred, and urea or guanidine hydrochloride at a concentration of about 6 to 8 M is preferred. A solution containing the protein denaturant can be an aqueous solution, a water-based solution, or a buffer containing the protein denaturant.

The solubilization can be performed at a temperature at which the solution components do not precipitate, and is preferably performed at room temperature. The time of the solubilization step can be, for example, 1 hour or longer, and is preferably within 12 hours. In general, 2 to 10 hours is desirable, and about 6 to 8 hours is desirable. When the time exceeds 12 hours, the viscosity increases, and when filtration is performed, the filtration may become difficult. The amount of protein denaturant to be added can be about 10 to 40 mL per E. coli pellet from 100 mL of a culture medium, and is preferably 15 to 35 mL.

In the method of one embodiment according to the present invention, the solubilized solution of transformant cells (lysate) can be purified using ion-exchange chromatography or immobilized metal affinity chromatography (IMAC). As the metal ion, copper, nickel, zinc, or cobalt is generally used, and as the carrier, nickel-nitrilotriacetic acid (Ni-NTA) agarose, Sepharose (registered trademark), and the like can be used. Purification by immobilized metal affinity chromatography can be performed under denaturing conditions, and it is preferable to use the same protein denaturant as that used in the solubilization step.

In the method of one embodiment according to the present invention, the elution solution and conditions can be appropriately selected according to the recombinant protein or the like. Elution may be performed using a competitive agent having a higher affinity for the metal than histidine, typically imidazole. The elution solution can be a solution (aqueous solution, water-based solution, or buffer) containing at least a competitive agent, for example, imidazole at a concentration of about 0.1 to 1 M, and the protein denaturant described above, for example, urea at a concentration of about 6 to 8 M. Elution can be performed under denaturing conditions and under weakly alkaline conditions (around pH 8.0) at a temperature at which the solution components do not precipitate, preferably at room temperature. Accordingly, specifically, for example, a solution of 50 mM tris hydrochloric acid (pH 8), 0.5 M sodium chloride, 8 M urea, and 0.5 M imidazole can be used.

The presence and degree of purification of the target protein in the recovered fraction can be confirmed by performing SDS-polyacrylamide gel electrophoresis (SDS-PAGE) or the like.

In the method according to one embodiment of the present invention, it is preferable to collect the fraction containing the target protein and remove unnecessary components used for purification by performing dialysis. The dialysis can be performed at a temperature at which the solution components do not precipitate, and is preferably performed at room temperature. The molecular weight cut-off value can be appropriately selected according to the molecular weight of the target protein.

The method according to one embodiment of the present invention may further include steps such as cell disruption (for example, ultrasonic treatment), washing, concentration, filtration, and modification, as necessary.

For example, in order to further increase the viscosity of the adhesive peptide portion, the purified recombinant protein may be subjected to a DOPA substitution step using tyrosinase. In the DOPA substitution step, tyrosine in the adhesive peptide portion is substituted with DOPA by reacting it in a reaction system containing borax, ascorbic acid, and tyrosinase. Borax serves to facilitate complex formation between protein systems, and ascorbic acid serves as an electron donor during DOPA substitution. Specifically, DOPA substitution can be performed by reacting the recombinant protein (1 mg/mL) at room temperature (within a range of 20 to 30°C) for 1 hour in a solution containing 20 mM borax, 25 mM ascorbic acid, and tyrosinase at a concentration in a range of 10 to 100 µg/mL.

The DOPA-substituted recombinant protein can be obtained in a viscous form by concentrating it 20-fold by ultrafiltration (pore size 3 kDa or 10 kDa), adding an equal volume of 1 M aqueous calcium chloride solution, and recovering it by centrifugation.

The recombinant protein may be subjected to any other modification according to the intended use, and for example, the adhesiveness may be enhanced by binding or adding an organic compound to the sequence of the adhesive peptide portion.

### <Preparation of Composition>

Using the recombinant protein according to one embodiment of the present invention, various compositions containing the recombinant protein together with any one or more optional components can be produced. For example, the recombinant protein of the present embodiment is biocompatible and has high safety, and therefore can be considered for use as a medical adhesive for surgical treatments and procedures, a pharmaceutical, a cosmetic-related quasi-drug, or a veterinary drug. In particular, the recombinant protein of the present embodiment can be used, in surgical procedures for artificial tissues (such as artificial blood vessels, artificial joints, and artificial bones), including dental implants, and organ or tissue transplantation between organisms (such as skin transplantation and retinal transplantation), for local migration and fixation of stem cells and the like, proliferation, and maintenance of an undifferentiated state, in addition to fixation of tissue cells, according to the effect of the functional peptide portion. Furthermore, the recombinant protein of the present embodiment can control cell adhesion, proliferation, and tissue regeneration particularly in the field of regenerative medicine including iPS cells.

The recombinant protein of the present embodiment can adhere cells in water without cytotoxicity. For this reason, the recombinant protein of the present embodiment is highly effective as a coating agent for adhering cultured cells, which can exist only in a culture medium, to plastic products (cell observation vessels such as culture dishes, chambers, and organs-on-a-chip), glass, and metals (such as titanium, gold, and magnets) in research in the biological field.

In addition, the recombinant protein of the present embodiment can have cell proliferation activity through fusion of the functional peptide portion, and can be used as a coating agent that enables not only cell culture and tissue culture but also cell proliferation, activation and maintenance of differentiation of cells and tissues, and local recruitment of cells. In addition, the recombinant protein of the present embodiment can also be used as a coated plastic vessel.

Furthermore, since the recombinant protein of the present embodiment can adhere to a living body even in water, it can be used in aquaculture technology. For example, the composition can be used as a composition for fixing a nucleus used in pearl cultivation inside a mother shell, or can be used as a composition for locally fixing fish eggs.

Such a composition can be a composition containing components such as known excipients, surfactants, oxidizing agents, and fillers that are selected according to the intended use and are applicable to living bodies. The surfactant may be any of a cationic surfactant, an anionic surfactant, a nonionic surfactant, an amphoteric surfactant, and the like. Examples of the oxidizing agent include tyrosinase, catechol oxidase, glutaraldehyde, formaldehyde, bis(sulfosuccinimidyl) suberate, 3,3'-dithiobis(sulfosuccinimidylpropionate), and hydrogen peroxide. Examples of the filler include collagen, hyaluronic acid, chondroitin sulfate, elastin, laminin, casein, hydroxyapatite, albumin, fibronectin, and fibrin.

### Examples

Hereinafter, the embodiments of the present invention will be specifically described below with reference to Examples, but the present invention is not limited to these Examples.

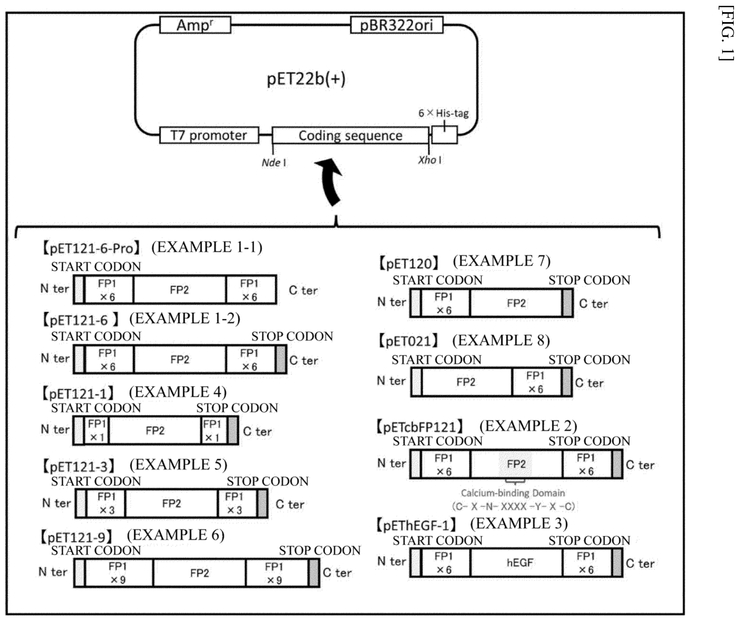

Table 1 and Fig. 1 show the names and configurations of the expression vector constructs prepared in the Examples, the amino acid sequences of the recombinant proteins, and the sequence ID numbers of nucleotide sequences of nucleic acids containing genes encoding the same. In Table 1 and Fig. 1, "FP1" represents a peptide sequence motif derived from FP1, "FP2" represents a peptide sequence derived from FP2, "cbFP2" represents a peptide sequence derived from FP2 including a calcium-binding domain, "hEGF" represents a peptide sequence derived from human EGF, and the number following "×" indicates the number of repeats of the motif.

**[Table 1]**

| Recombinant protein/ | Structure | Name of vector | Example | SEQ ID NO: | | |
|---|---|---|---|---|---|---|
| Name of gene | (from N-terminus to C-terminus/from 5' to 3') | | (representative examples are given, but not limitedthereto) | | | |
| | | | | Synthetic fragment | Insert fragment | Protein |
| FP121-1 | FP1(×1)-FP2(×1)-FP1(×1) | pET121-1 | 4, 11 | 35 | 36 | 68 |
| FP121-3 | FP1(×3)-FP2(×1)-FP1(×3) | pET121-3 | 5, 11, 17~20, 23 | 39 | 40 | 69 |
| FP121-6 | FP1(×6)-FP2(×1)-FP1(×6)-Histag | pET121-6-Pro | 1, 10, 15-18 | 25 | 26 | 64 |
| (prototype) | | | | | | |
| FP121-6 | FP1(×6)-FP2(×1)-FP1(×6)-Histag | pET121-6-Pro | 1, 10, 14-16, 23 | 25 | 26 | 64 |
| (prototype) | | | | | | |
| DOPA modification | | | | | | |
| FP121-6 | FP1(×6)-FP2(×1)-FP1(×6) | pET121-6 | 1, 11, 17~20, 23, 28, 29 | 29 | 30 | 65 |
| FP121-9 | FP1(×9)-FP2(×1)-FP1(×9) | pET121-9 | 6, 11, 17, 18, 23 | 43 | 57 | 70 |
| FP120 | FP1(×6)-FP2(×1) | pET120 | 7, 12, 19, 20 | 58 | 60 | 71 |
| FP021 | FP2(×1)-FP1(×6) | pET021 | 8, 12, 19, 20 | 61 | 63 | 72 |
| cbFP121-6 | FP1(×6)-cbFP2(×1)-FP1(×6) | pETcb121 | 2 | 31 | 32 | 66 |
| hEGF-FP1-6 | FP1(×6)-hEGF-FP1(×6) | pEThEGF-1 | 3, 21, 22 | 33 | 34 | 67 |
| hNGF-FP1-6 | FP1(×6)-hNGF-FP1(×6) | pEThNGF-1 | 25, 27~29 | 76 | | 75 |
| hbFGF-FP1-6 | FP1(×6)-hbFGF-FP1(×6) | pEThbFGF-1 | 26~27 | 78 | | 77 |
| FP151 | | | 15, 16, 23, 24, 28, 29 | | 74 | 73 |

Table 2 shows the correspondence of the configurations of the amino acid sequences of the adhesive peptide portions of the insert fragments.

**[Table 2]**

| SEQ ID NO: | N-terminus=> C-terminus (SEQ ID NO:) |
|---|---|
| **18** | **(13) (14) (15) (16) (14) (14)** |
| **19** | **(13) (14) (15) (16) (14) (15)** |
| **20** | **(16) (14) (14)** |
| **21** | **(13) (14) (15)** |
| **22** | **(13) (14) (15) (13) (14) (15) (16) (14) (14)** |
| **23** | **(13) (14) (15) (16) (14) (14) (16) (14) (14)** |

### 1. Construction of Expression Vector

### <Example 1: Construction of Gene and Vector for Production of FP121-6 Recombinant Protein>

### [1-1: Construction of Gene and Vector of FP121-6 (Prototype)]

A fragment containing the FP121-6 (prototype) gene was synthesized so that an NdeI restriction enzyme site was positioned on the 5' end side and an XhoI restriction enzyme site was positioned on the 3' end side of the FP121-6 (prototype) gene sequence (SEQ ID NO: 25). This was digested with NdeI and XhoI and inserted into a pET22b(+) vector (Novagen, USA) digested with the same enzymes, thereby producing the pET121-6-Pro vector. The sequence ID number of the inserted fragment is SEQ ID NO: 26. Since the FP121-6 (prototype) gene does not contain a stop codon within the structural gene, this recombinant protein is expressed in a form in which a 6×His-tag derived from the vector is fused to the C-terminal side (SEQ ID NO: 64).

### [1-2: Construction of Gene and Vector of FP121-6]

A fragment containing the FP121-6 gene including a stop codon (SEQ ID NO: 29) was amplified using the pET121-6-Pro vector as a template and a primer of SEQ ID NO: 27 (5'-TAATACGACTCACTATAGGG-3') and a primer of SEQ ID NO: 28 (5'-TTTCTCGAGTTACTTGTACGTCGGCGGGTAAGACGGTTTTG-3'). Thereafter, the amplified product was inserted into the pET22b(+) vector using the NdeI and XhoI restriction enzyme sites thereof in the same manner as described above, thereby producing a pET121-6 vector. The sequence ID number of the inserted fragment is SEQ ID NO: 30. From the pET121-6 vector, the recombinant protein FP121-6 (SEQ ID NO: 65) is expressed via a protein expression system through the pET system using a T7 promoter.

### <Example 2: Construction of Gene and Vector for Recombinant Production of cbFP121-6>

A fragment containing the cbFP121-6 gene was synthesized so that an NdeI restriction enzyme site was positioned on the 5' end side and an XhoI restriction enzyme site was positioned on the 3' end side of the cbFP121-6 gene sequence (SEQ ID NO: 31). This was inserted into a pET22b(+) vector in the same manner as described above, thereby producing a pETcb121 vector. The sequence ID number of the inserted fragment is SEQ ID NO: 32. From the pETcb121 vector, the recombinant protein cbFP121-6 (SEQ ID NO: 66) is expressed via a protein expression system through the pET system.

### <Example 3: Construction of Gene and Vector for Recombinant Production of hEGF-FP1-6>

A fragment containing the hEGF-FP1-6 gene was synthesized so that an NdeI restriction enzyme site was positioned on the 5' end side and an XhoI restriction enzyme site was positioned on the 3' end side of the hEGF-FP1-6 gene sequence (SEQ ID NO: 33). This was inserted into a pET22b(+) vector in the same manner as described above, thereby producing a pEThEGF-1 vector. The sequence ID number of the inserted fragment is SEQ ID NO: 34. From the pEThEGF-1 vector, the recombinant protein hEGF-FP1-6 (SEQ ID NO: 67) is expressed via a protein expression system through the pET system.

### <Example 4: Construction of Gene and Vector for Recombinant Production of FP121-1>

A fragment containing the FP121-1 gene (SEQ ID NO: 35) was amplified using the pET121-6 vector as a template and a primer of SEQ ID NO: 37 (5'-AAACATATGGCCAAGCCGTCCTACCCGCCTACCTATAAACCCGTGAAC-3') and a primer of SEQ ID NO: 38 (5'-TTTCTCGAGTTACTTATAGGTTGACGGGTAGGTCGGCTTTGCATTGC-3'). Thereafter, the amplified product was inserted into the pET22b(+) vector using the NdeI and XhoI restriction enzyme sites in the same manner as described above, thereby producing a pET121-1 vector. The sequence ID number of the inserted fragment is SEQ ID NO: 36. From the pET121-1 vector, the recombinant protein FP121-1 (SEQ ID NO: 68) is expressed via a protein expression system through the pET system.

### <Example 5: Construction of Gene and Vector for Recombinant Production of FP121-3>

A fragment containing the FP121-3 gene (SEQ ID NO: 39) was amplified using the pET121-6 vector as a template and a primer of SEQ ID NO: 41 (5'-AAACATATGGCTAAGAGCAGCTACCCGCCAACGTACAAAGCG-3') and a primer of SEQ ID NO: 42 (5'-TTTCTCGAGTTACTTGTAGGTCGACGGATAGCTCGGC-3'). Thereafter, the amplified product was inserted into the pET22b(+) vector using the NdeI and XhoI restriction enzyme sites in the same manner as described above, thereby producing a pET121-3 vector. The sequence ID number of the inserted fragment is SEQ ID NO: 40. From the pET121-3 vector, the recombinant protein FP121-3 (SEQ ID NO: 69) is expressed via a protein expression system through the pET system.

### <Example 6: Construction of Gene and Vector for Recombinant Production of FP121-9>

### [6-1. Preparation of Gene Fragment]

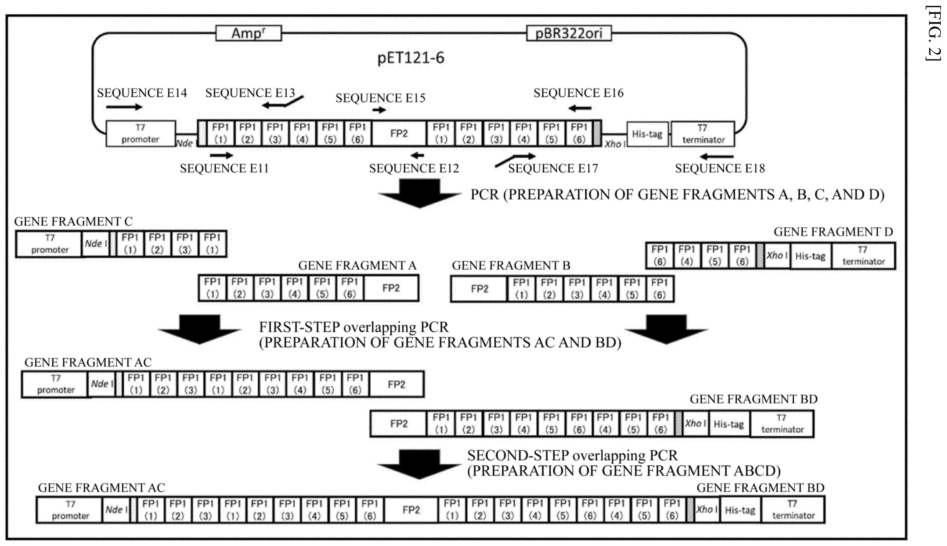

A fragment containing the FP121-9 gene (SEQ ID NO: 43) was prepared by separately producing gene fragments A (SEQ ID NO: 44), B (SEQ ID NO: 45), C (SEQ ID NO: 46), and D (SEQ ID NO: 47), as illustrated in Fig. 2, and linking the respective gene fragments by two-step Overlapping PCR.

A gene fragment A (SEQ ID NO: 44) was amplified from the pET121-6 vector using a primer of SEQ ID NO: 48 (5'-GCCAAGCCGACGTACCCCTCAACTTACAAGGCGAAG-3') and a primer of SEQ ID NO: 49 (5'-CATTGCAGTTATAGCCGTAATAACC-3'). A gene fragment B (SEQ ID NO: 45) was amplified from the pET121-6 vector using a primer of SEQ ID NO: 50 (5'-CCCGTGAACCCGTGTCTG-3') and a primer of SEQ ID NO: 51 (5'-CTTTGTATGTCGGCGGGTAAGACGGTTTTGC-3').

A gene fragment C (SEQ ID NO: 46) was amplified from the pET121-6 vector using a primer of SEQ ID NO: 52 (5'-CTTCGCCTTGTAAGTTGAGGGGTACGTCGGCTTGGCTTTGTAGGTGGACGGG TAAGAC-3') and a primer of SEQ ID NO: 27 (5'-TAATACGACTCACTATAGGG-3'). A sequence complementary to the C-terminal side of gene fragment A was added to the N-terminal side of the amplified gene fragment C. A gene fragment D (SEQ ID NO: 47) was amplified from the pET121-6 vector using a primer of SEQ ID NO: 53 (5'-GCAAAACCGTCTTACCCGCCGACATACAAAGCGAAAAGCAGCTACCCACC-3') and a primer of SEQ ID NO: 54 (5'-ATGCTAGTTATTGCTCAGCGG-3'). A sequence complementary to the C-terminal side of gene fragment B was added to the N-terminal side of the amplified gene fragment D.

A gene fragment AC (SEQ ID NO: 55) was produced by linking the gene fragment A and the gene fragment C prepared in the above step by Overlapping PCR, and re-amplifying them using a primer of SEQ ID NO: 49 (5'-CATTGCAGTTATAGCCGTAATAACC-3') and a primer of SEQ ID NO: 27 (5'-TAATACGACTCACTATAGGG-3'). A gene fragment BD (SEQ ID NO: 56) was produced by linking the gene fragment B and the gene fragment D prepared in the above step by Overlapping PCR, and re-amplifying them using a primer of SEQ ID NO: 50 (5'-CCCGTGAACCCGTGTCTG-3') and a primer of SEQ ID NO: 54 (5'-ATGCTAGTTATTGCTCAGCGG-3').

The gene fragment ABCD (SEQ ID NO: 43) was prepared by linking the gene fragment AC and the gene fragment BD prepared in the above step by Overlapping PCR.

### [6-2. Construction of Vector]

A fragment containing the FP121-9 gene (SEQ ID NO: 43) was amplified using the gene fragment ABCD prepared in Example 6-1 as a template and a primer of SEQ ID NO: 27 (5'-TAATACGACTCACTATAGGG-3') and a primer of SEQ ID NO: 54 (5'-ATGCTAGTTATTGCTCAGCGG-3'). Thereafter, the amplified product was inserted into the pET22b(+) vector using the NdeI and XhoI restriction enzyme sites thereof, thereby producing a pET 121-9 vector. The sequence ID number of the inserted fragment is SEQ ID NO: 57. From the pET121-9 vector, the recombinant protein FP121-9 (SEQ ID NO: 70) is expressed.

### <Example 7: Construction of Gene Sequence and Vector for Recombinant Production of FP120>

A fragment containing the FP120 gene (SEQ ID NO: 58) was amplified from the pET121-6 vector using a primer of SEQ ID NO: 27 (5'-TAATACGACTCACTATAGGG-3') and a primer of SEQ ID NO: 59 (5'-TTTCTCGAGTTAATTGCAGTTATAGCCGTAATAACC-3'). Thereafter, the amplified product was inserted into the pET22b(+) vector using the NdeI and XhoI restriction enzyme sites thereof, thereby producing a pET120 vector. The sequence ID number of the inserted fragment is SEQ ID NO: 60. From the pET120 vector, the recombinant protein FP120 (SEQ ID NO: 71) is expressed.

### <Example 8: Construction of Gene Sequence and Vector for Recombinant Production of FP021>

A fragment containing the FP021 gene (SEQ ID NO: 61) was amplified from the pET121-6 vector using a primer of SEQ ID NO: 62 (5'-AAACATATGCCCGTGAACCCGTGTCTG-3') and a primer of SEQ ID NO: 54 (5'-ATGCTAGTTATTGCTCAGCGG-3'). Thereafter, the amplified product was inserted into the pET22b(+) vector using the NdeI and XhoI restriction enzyme sites thereof, thereby producing a pET021 vector. The sequence ID number of the inserted fragment is SEQ ID NO: 63. From the pET021 vector, the recombinant protein FP021 (SEQ ID NO: 72) is expressed.

### 2. Production of Transformants, and Expression, Purification, and Modification of Recombinant Proteins

### <Example 9: Production of Transformants Producing Recombinant Proteins>

As a cell for protein expression, E. coli BL21(DE3) (fhuA2 "lon" ompT gal (λ DE3) "dcm" ΔhsdS λ DE3 = λ sBamHIo ΔEcoRI-B int::(lacI::PlacUV5::T7 gene1) i21 Δnin5) was used. Into these cells, the expression vectors pET121-1, pET121-3, pET121-6, pET121-6-Pro, pET121-9, pET120, pET021, pETcb121, and pEThEGF-1 of Examples 1 to 8 were introduced, respectively, using an electroporation method.

These transformants (E. coli BL21/pET121-1, E. coli BL21/pET121-3, E. coli BL21/pET121-6, E. coli BL21/pET121-6-Pro, E. coli BL21/pET121-9, E. coli BL21/pET120, E. coli BL21/pET021, E. coli BL21/pETcb121, and E. coli BL21/pEThEGF-1) were obtained through a selection process using ampicillin (Nacalai, Japan).

### <Example 10: Expression and Purification of Recombinant Protein FP121-6 (Prototype)>

### [10-1. Culture of Transformants]

The transformant E. coli BL21/pET121-6-Pro obtained in Example 9 was cultured in 2×YT medium. Specifically, a bacterial colony was pre-cultured for 5 to 16 hours in 5 mL of 2×YT medium (containing 100 µg/mL ampicillin) in a sterile 50 mL tube. 4 mL of this pre-culture medium was inoculated into 400 mL of 2×YT medium in a 2 L baffled flask, and the main culture was performed. Unless otherwise specified, all cultures were performed at 37°C. When the absorbance (OD600) of the culture medium at 600 nm reached around 1.5, IPTG was added at a final concentration of 1 mM to induce the expression of the recombinant protein. The culture medium after completion of the culture step was centrifuged at 3,000 × g for 15 minutes to collect the cell pellet. This was stored at -80°C until use in the following experiment.

### [10-2. Extraction of Recombinant Protein]

To 1 g of the cells collected in Example 10-1, 10 mL of 25% acetic acid was added to extract the recombinant protein, and the resulting mixture was centrifuged at 16,000 × g at 4°C for 15 minutes to obtain a recombinant protein extract. The recombinant protein extract solution was clarified with a 0.45 µm filter, and then an equal volume of 500 mM sodium acetate was added to neutralize the pH to a range of 3 to 4. Thereafter, the recombinant protein was separated and purified by cation exchange chromatography as follows.

### [10-3. Purification of Recombinant Protein]

As a chromatography carrier, "CM Sepharose (registered trademark) Fast Flow" (Cytiva) was used. The column was filled with 6 mL of the carrier, and then equilibrated by passing an equilibration buffer (50 mM sodium acetate buffer (pH 5.5), 250 mM sodium chloride). After passing the recombinant protein solution prepared in Example 10-2 through this column, the column was washed by passing through 5 column volumes (CV) or more of the equilibration buffer. The recombinant protein was eluted using 50 mM sodium acetate buffer containing 750 mM sodium chloride. The elution fractions were collected in portions of 5 mL each.

Samples of each elution fraction during the purification step were subjected to absorbance measurement at a wavelength of 280 nm (A280) and SDS-PAGE analysis to confirm the recombinant protein. In the SDS-PAGE analysis, a 15% SDS-PAGE gel was used. 7.5 µL of each sample was mixed with an equal volume of electrophoresis sample buffer (0.125 M Tris-HCl, 4 (w/v)% SDS, 20 (v/v)% glycerol, 0.01 (w/v)% BPB, and 10 (v/v)% 2-ME) to prepare an electrophoresis sample. Electrophoresis was performed at 250 V and 75 mA, and the recombinant protein was visualized by staining with Coomassie Blue.

**[Table 3]**

| Lane | Sample | Sample amount | A280 |
|---|---|---|---|
| **M** | **Protein Molecular Weight Marker (Broad)** | **5 µL** | - |
| **1** | Acetic acid extract (after neutralization) | **7.5 µL** | **0.381** |
| **2** | Flow-through fraction | **7.5 µL** | **0.044** |
| **3** | Wash fraction | **7.5 µL** | **0.000** |
| **4** | Elution fraction 1 | **7.5 µL** | **0.001** |
| **5** | Elution fraction 2 | **7.5** µL | **-0.001** |
| **6** | Elution fraction 3 | **7.5 µL** | **2.154** |
| **7** | Elution fraction 4 | **7.5 µL** | **4.353** |
| **8** | Elution fraction 5 | **7.5 µL** | **1.733** |

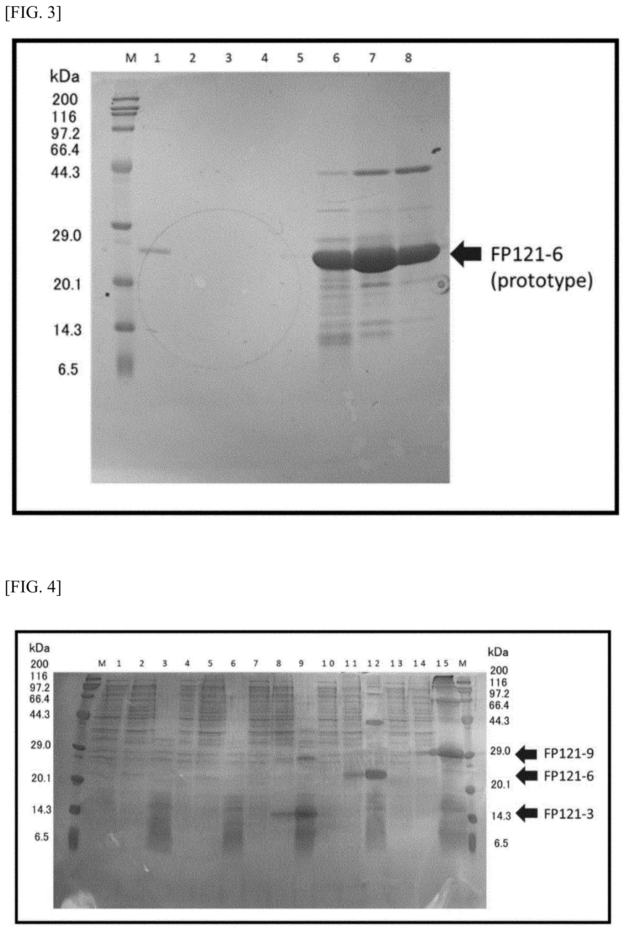

The results of the SDS-PAGE are illustrated in Fig. 3. A high concentration of protein was detected in the samples of elution fractions 3 to 5 (lanes 6 to 8). Thus, it was confirmed that the recombinant protein of the present invention can be purified by ion-exchange chromatography. Note that the reason why FP121-6 (prototype) having a theoretical molecular weight of 18kDa is detected as a 23 kDa protein in Fig. 3 is that FP121-6 (prototype) is a basic protein containing 18% lysine, which is a basic amino acid, which causes a lower electrophoretic mobility in SDS-PAGE analysis, resulting in an apparently higher molecular weight.

The elution fractions containing the recombinant protein were combined and desalted by dialysis ("Spectra/Por1", REPLIGEN, USA) in a 0.5% acetic acid solution at room temperature. The protein concentration of the desalted recombinant protein solution was measured using "Qubit Protein and Protein Broad Range (BR) Assay Kits" (Invitrogen, USA). The yield of the purified recombinant protein prepared was about 15.6 mg.

### <Example 11: Expression of Recombinant Proteins FP121-1, FP121-3, FP121-6, and FP121-9>

### [11-1. Culture of Transformants]

The transformants (E. coli BL21/pET121-1, E coli BL21/pET121-3, E. coli BL21/pET121-6, and E. coli BL21/pET121-9) obtained in Example 9 were each cultured in 2×YT medium. As a negative control, E. coli BL21/pET22b transformed with a vector containing no inserted gene was cultured under the same conditions.

Each bacterial colony was pre-cultured in the same manner as in Example 10-1. 1 mL of this pre-culture medium was inoculated into a 250 mL baffled flask containing 50 mL of 2×YT medium, and the main culture was performed. When the OD600 of the culture medium reached about 0.4 to 1.0, IPTG was added at a final concentration of 1 mM to induce the expression of the recombinant protein.

E. coli during the culture step was sampled in an amount of about 1 mL each before and after IPTG addition for expression analysis experiments. The culture medium after completion of the culture step was centrifuged at 3,000 × g for 15 minutes to collect the cell pellet. This was stored at -80°C until use in the following experiment.

### [11-2. Expression Analysis and Extraction Confirmation of Recombinant Proteins]

The recombinant protein was extracted by adding 10 mL of 25% acetic acid to 1 g of the cells collected in Example 11-1. The insoluble fraction in the extract was removed by centrifugation at 16,000 × g at 4°C for 15 minutes. To remove the extraction solvent from the recombinant protein extract, the solution was clarified using a 0.45 µm filter and then lyophilized to obtain a powder. The lyophilized recombinant protein was re-dissolved using 50 mM sodium acetate buffer (pH 5.5).

The concentration of E. coli in the culture sampled during the culture step was adjusted to OD600 = 1.0 ± 0.1, and mixed with an equal volume of electrophoresis sample buffer to prepare an E. coli lysate. Each of the E. coli lysate and the re-dissolved extract solution samples was subjected to electrophoresis and staining using a 17.5% SDS-PAGE gel in the same manner as in Example 10-3.

**[Table 4]**

| Lane | Transformant | Condition | Sample amount |
|---|---|---|---|
| M | Protein Molecular Weight Marker (Broad) | - | 5µL |
| 1 | E.coli BL21/pET22b (negative control) | Before induction | 10 µL |
| 2 | | After induction | 10 µL |
| 3 | | After re-dissolution | 10 µL |
| 4 | E.coli BL21/pET121-1 | Before induction | 10 µL |
| 5 | | After induction | 10 µL |
| 6 | | After re-dissolution | 10 µL |
| 7 | E.coli BL21/pET121-3 | Before induction | 10 µL |
| 8 | | After induction | 10 µL |
| 9 | | After re-dissolution | 10 µL |
| 10 | E.coli BL21/pET121-6 | Before induction | 10 µL |
| 11 | | After induction | 10 µL |
| 12 | | After re-dissolution | 10 µL |
| 13 | E.coli BL21/pET121-9 | Before induction | 10 µL |
| 14 | | After induction | 10 µL |
| 15 | | After re-dissolution | 10 µL |
| M | Protein Molecular Weight Marker (Broad) | - | 5 µL |

The results of the SDS-PAGE are illustrated in Fig. 4. In the induced cultures of E. coli BL21/pET121-3, E. coli BL21/pET121-6, and E. coli BL21/pET121-9 (lanes 8, 11, and 14, respectively) and in the re-dissolved extract solutions, which are the protein fractions thereof (lanes 9, 12, and 15, respectively), the expression of each of FP121-3 (about 15 kDa), FP121-6 (about 23 kDa), and FP121-9 (about 30 kDa) was confirmed. FP121-1 was expressed at a low level and was not visualized by Coomassie Blue.

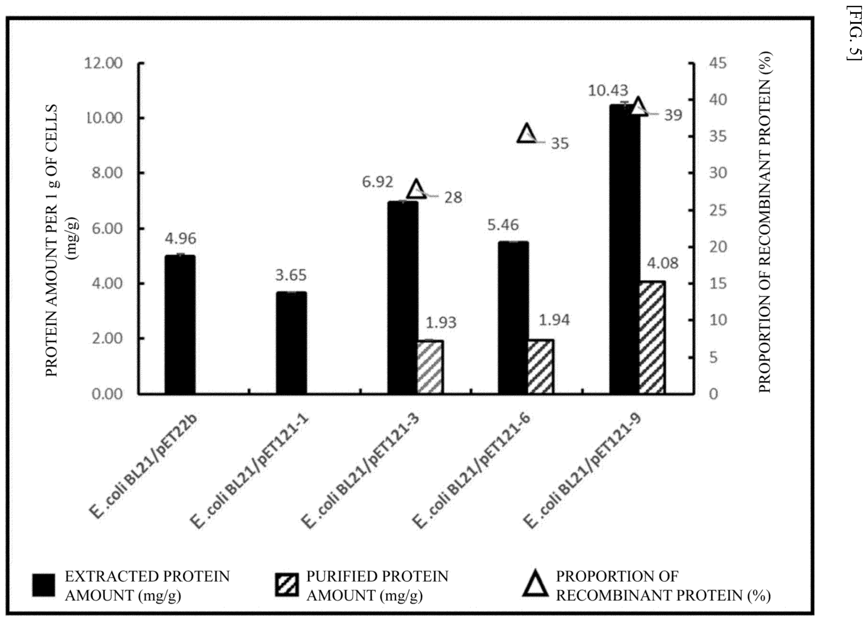

The protein concentration in the re-dissolved extract was measured by the same method as in Example 10-3, and the yield was calculated. The results are illustrated in Fig. 5. The mass of extracted protein per 1 g of cells was 10.43 mg/g for FP121-9, 5.46 mg/g for FP121-6, 6.92 mg/g for FP121-3, 3.65 mg/g for FP121-1, and 4.96 mg/g for the negative control.

### [11-3. Purification of Recombinant Protein]

FP121-3, FP121-6, and FP121-9 prepared in Example 11-2 were purified. Note that FP121-1, as shown in the SDS-PAGE results of Example 11-2, exhibited a low expression level and therefore was not subjected to the purification step.

Purification was performed by cation exchange chromatography in the same manner as in Example 10-3. However, 2 mL of carrier and, as an equilibration buffer, a 50 mM sodium acetate buffer (pH 5.5) and 50 mM sodium chloride were used, and, after passing each of the recombinant proteins FP121-3, FP121-6, and FP121-9 prepared in Example 11-2 through the column, the proteins were eluted sequentially using 50 mM sodium acetate buffer containing 250, 500, 750, or 1,000 mM sodium chloride.

For each sample of the elution fractions, A280 measurement and SDS-PAGE analysis were performed to confirm the recombinant proteins. In the SDS-PAGE analysis, 10 µL of each sample was used, with a 17.5% SDS-PAGE gel for FP121-3 and a 15% SDS-PAGE gel for FP121-6 and FP121-9, respectively, and the other conditions were the same as in Example 10-3.

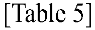

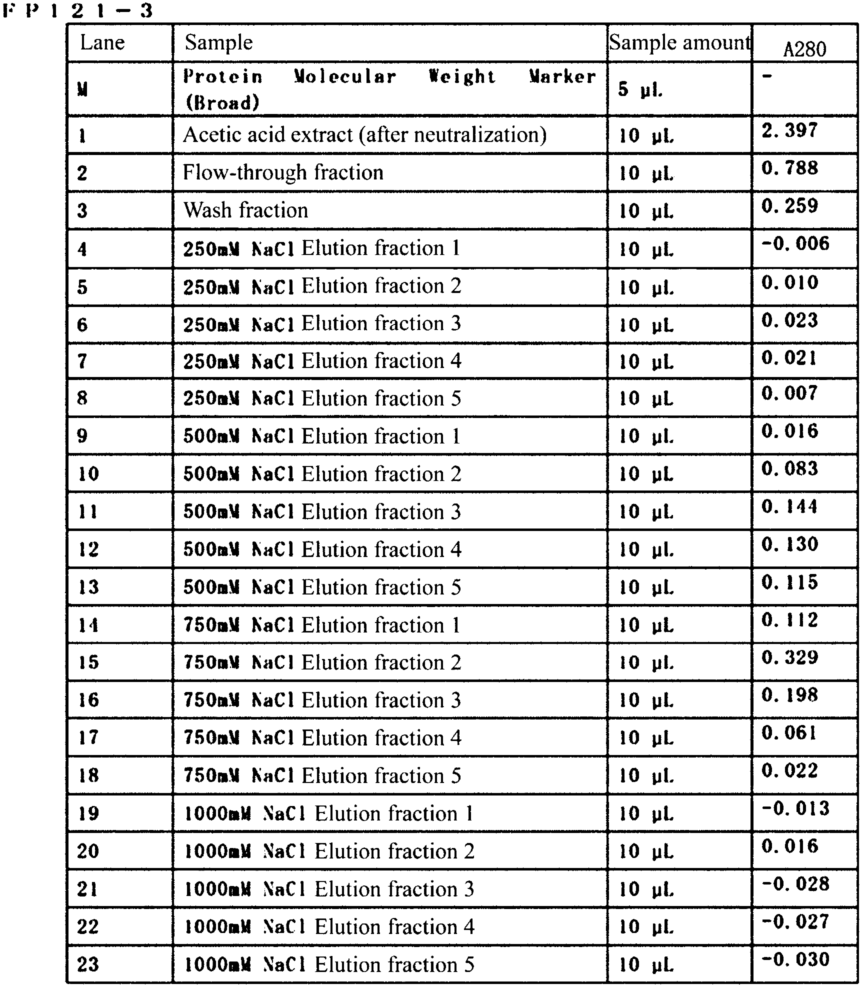

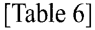

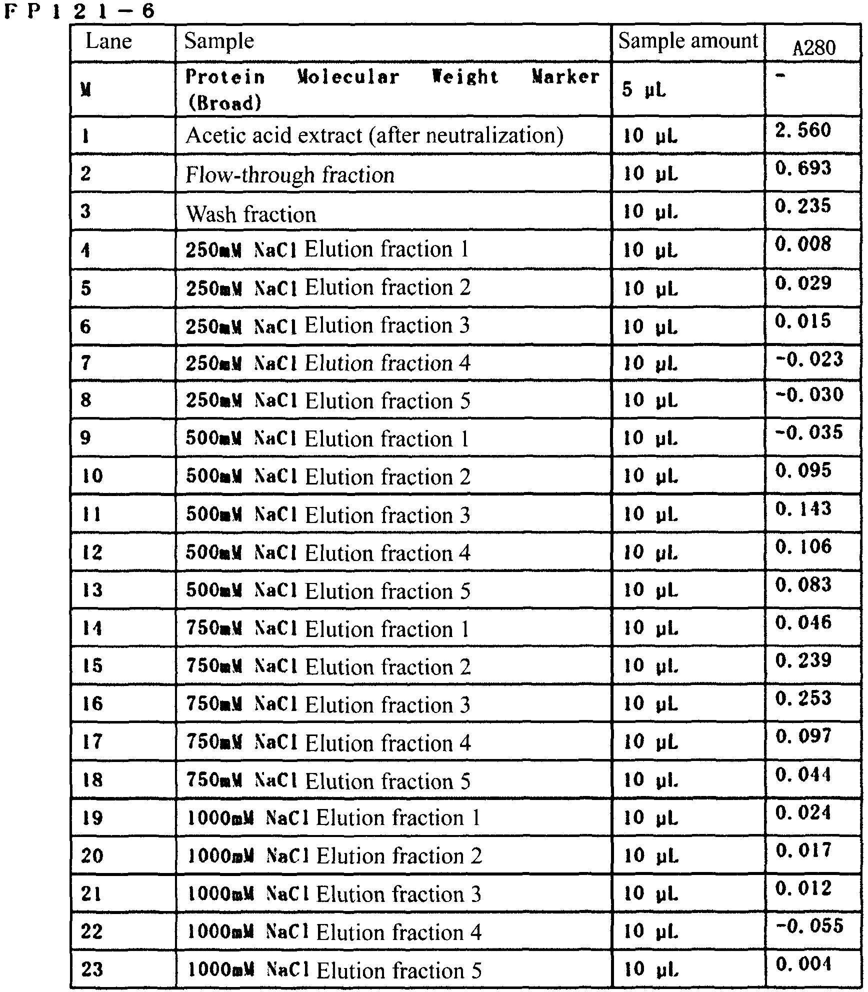

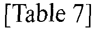

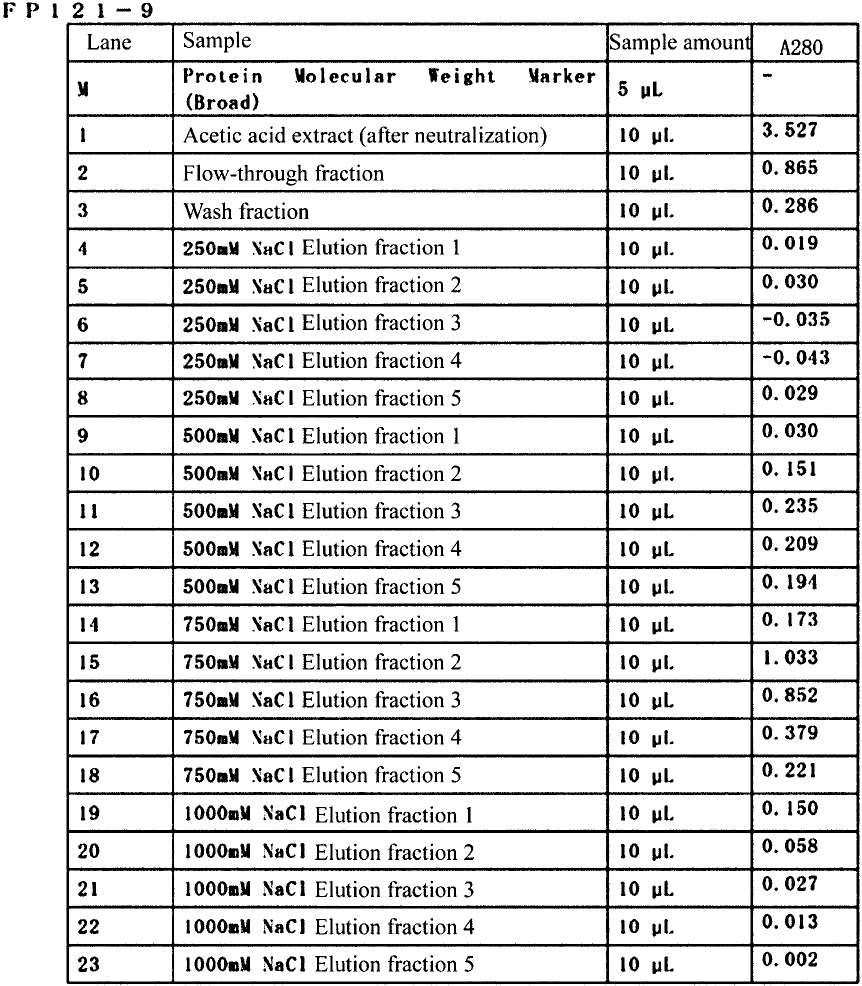

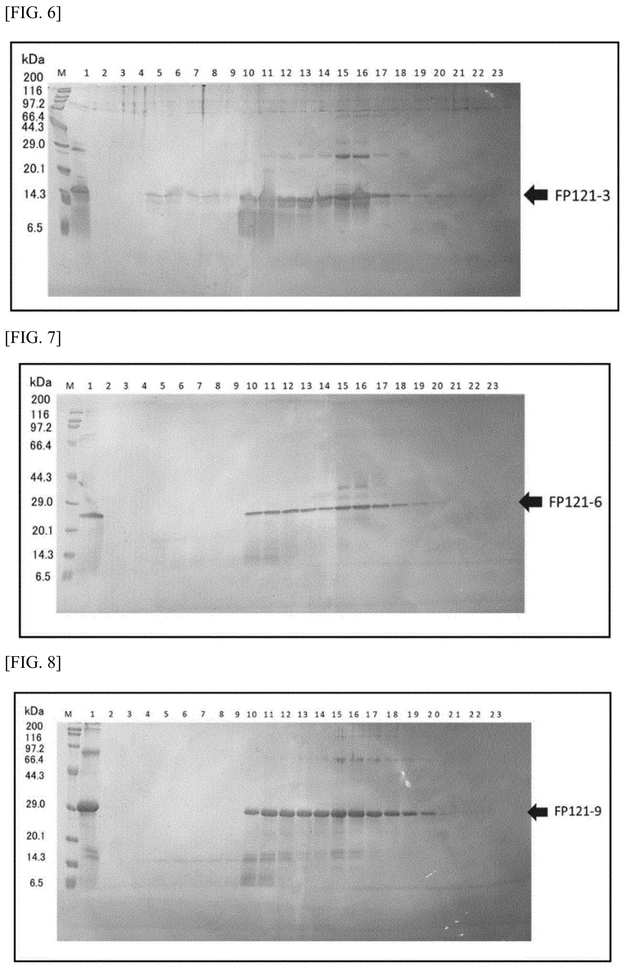

The results of the SDS-PAGE are illustrated in Figs. 6, 7, and 8. High concentrations of protein were detected in the samples of the 500 mM and 750 mM NaCl elution fractions (lanes 10 to 17 for FP121-3 and lanes 10 to 18 for FP121-6) and in the samples of the 500 mM, 750 mM, and 1,000 mM NaCl elution fractions (lanes 10 to 20) for FP121-9.

The elution fractions containing each recombinant protein were combined, and desalting was performed in the same manner as in Example 10-3. The concentration of each recombinant protein was measured in the same manner as in Example 10-3, and the mass of purified protein and the amount of purified protein per 1 g of cells were calculated from the volume of the recovered solution and the amount of cells.

The results are illustrated in Fig. 5. The masses of purified protein were 1.26 mg for FP121-3, 1.11 mg for FP121-6, and 2.29 mg for FP121-9. The masses of purified protein per 1 g of cells were 1.93 mg/g for FP121-3, 1.94 mg/g for FP121-6, and 4.08 mg/g for FP121-9.

The proportion (%) of recombinant protein contained in the extracted protein was calculated using the calculation formula "(amount of purified protein per 1 g of cells/amount of extracted protein per 1 g of cells) × 100", and the result showed that FP121-3 was 28%, FP121-6 was 35%, and FP121-9 was 39%.

### <Example 12: Expression of Recombinant Proteins FP120 and FP021>

### [12-1. Culture of Transformants]

The transformants E coli BL21/pET120 and E. coli BL21/pET021 obtained in Example 9 were cultured in the same manner as in Example 11-1, sampled before and after the addition of IPTG, and, after completion of the culture step, the cell pellets were collected and stored at -80°C. As controls, E. coli BL21/pET22b (negative control) and E. coli BL21/pET121-6 (positive control) were used.

### [12-2. Expression Analysis and Extraction Confirmation of Recombinant Proteins]

The expression analysis and extraction confirmation of the recombinant proteins were performed in the same manner as in Example 11-2.

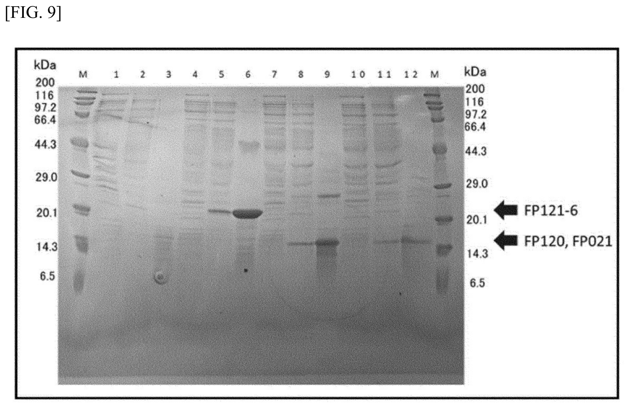

The results are illustrated in Fig. 9. From the results of the SDS-PAGE analysis, the expression and extraction of FP120 (about 15 kDa) from the induced E. coli BL21/pET120 and FP210 (about 15 kDa) from the induced E. coli BL21/pET021 were confirmed.

**[Table 8]**

| FP120 and FP021 | | | |
|---|---|---|---|
| Lane | Transformant | Condition | Sample amount |
| M | Protein Molecular Weight Marker (Broad) | - | 5µL |
| 1 | E.coli BL21/pET22b (negative control) | Before induction | 10 µL |
| 2 | | After induction | 10 µL |
| 3 | | After re-dissolution | 10 µL |
| 4 | E.coli BL21/pET121-6 | Before induction | 10 µL |
| 5 | | After induction | 10 µL |
| 6 | | After re-dissolution | 10 µL |
| 7 | E.coli B1-21/pET120 | Before induction | 10 µL |
| 8 | | After induction | 10 µL |
| 9 | | After re-dissolution | 10 µL |
| 10 | E.coli B121/pET021 | Before induction | 10 µL |
| 11 | | After induction | 10 µL |
| 12 | | After re-dissolution | 10 µL |
| M | Protein Molecular Weight Marker (Broad) | - | 5 µL |

The total amount of extracted protein was calculated in the same manner as in Example 11-2.

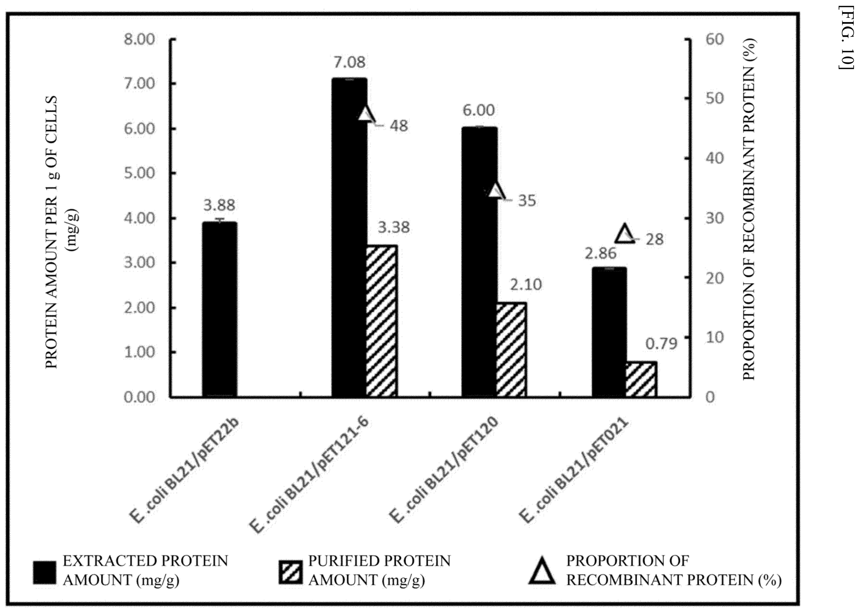

The results are illustrated in Fig. 10. The amount of protein extracted per 1 g of cells was 6.00 mg/g for FP120, 2.86 mg/g for FP021, 7.08 mg/g for FP121-6 (positive control), and 3.88 mg/g for the negative control.

### [12-3. Purification of Recombinant Protein]

FP120, FP021, and FP121-6 (positive control) were each purified in the same manner as in Example 11-3. For each sample in the purification step, A280 measurement and SDS-PAGE analysis were performed to confirm the recombinant proteins. For SDS-PAGE analysis of FP120 and FP021, a 17.5% SDS-PAGE gel was used. The other procedures were performed under the same conditions as in Example 10-3.

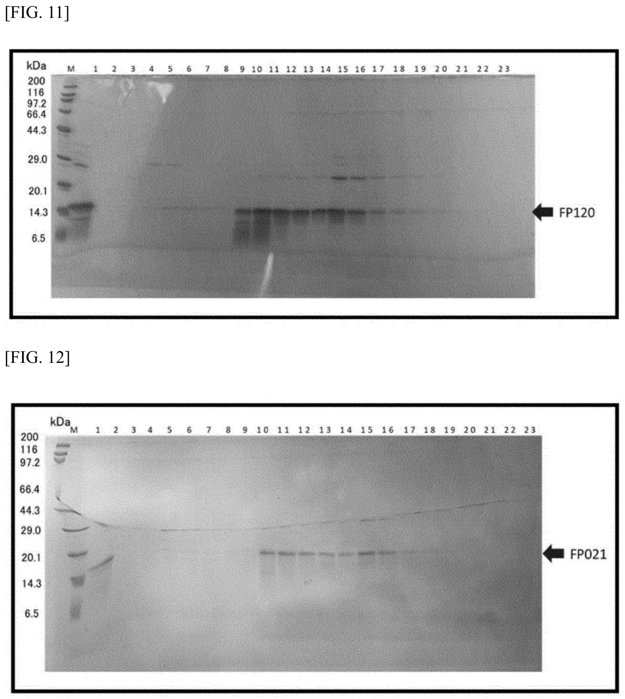

The results of the SDS-PAGE are illustrated in Figs. 11 and 12.

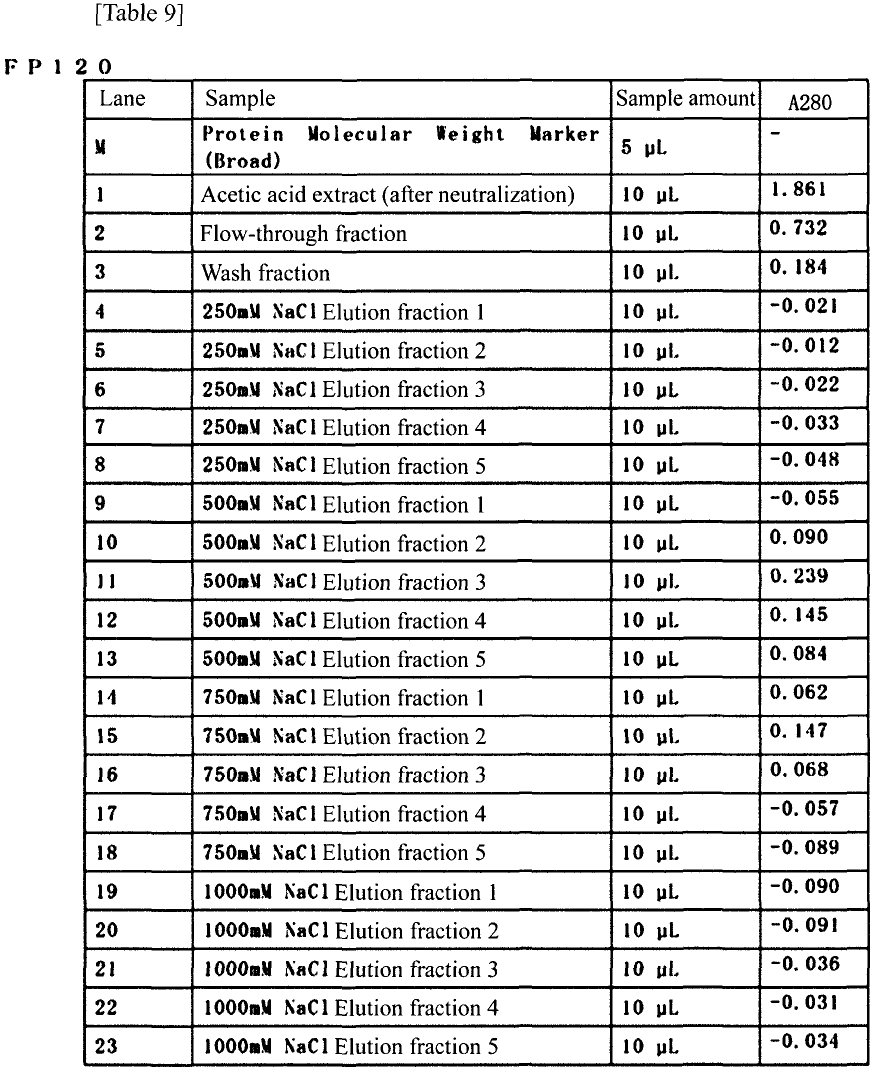

**[Table 10]**

| **FP021** | | | |
|---|---|---|---|
| Lane | Sample | Sample amount | A280 |
| **M** | **Protein Molecular Weight Marker (Broad)** | **5 µL** | - |
| **1** | Acetic acid extract (after neutralization) | **10 µL** | **1.241** |
| **2** | Flow-through fraction | **10 µL** | **0.625** |
| **3** | Wash fraction | **10 µL** | **0.184** |
| **4** | **250mM NaCl** Elution fraction 1 | **10 µL** | **-0.020** |
| **5** | **250mM NaCl** Elution fraction 2 | **10 µL** | **-0.008** |
| **6** | **250mM NaCl** Elution fraction 3 | **10 µL** | **-0.011** |
| **7** | **250mM NaCl** Elution fraction 4 | **10 µL** | **-0.023** |
| **8** | **250mM NaCl** Elution fraction 5 | **10 µL** | **-0.034** |
| **9** | **500mM NaCl** Elution fraction 1 | **10 µL** | **-0.031** |
| **10** | **500mM NaCl** Elution fraction 2 | **10 µL** | **0.042** |
| 11 | 500mM NaCl Elution fraction 3 | **10 µL** | **0.024** |
| 12 | 500mM **NaCl** Elution fraction 4 | **10 µL** | **0.019** |
| **13** | **500mM NaCl** Elution fraction 5 | **10 µL** | -0.042 |
| **14** | **750mM NaCl** Elution fraction 1 | **10 µL** | **-0.057** |
| **15** | **750mM NaCl** Elution fraction 2 | **10 µL** | **0.020** |
| **16** | **750mM NaCl** Elution fraction 3 | **10 µL** | **-0.058** |
| **17** | **750mM NaCl** Elution fraction 4 | **10 µL** | **-0.073** |
| **18** | **750mM NaCl** Elution fraction 5 | **10 µL** | **-0.079** |
| **19** | **1000mM NaCl** Elution fraction 1 | **10 µL** | **-0.079** |
| **20** | **1000mM NaCl** Elution fraction 2 | **10 µL** | **-0.070** |
| **21** | **1000mM NaCl** Elution fraction 3 | **10 µL** | **-0.019** |
| **22** | **1000mM NaCl** Elution fraction 4 | **10 µL** | **-0.032** |
| **23** | **1000mM NaCl** Elution fraction 5 | **10 µL** | **-0.105** |

The elution fractions containing each recombinant protein were combined, and desalting was performed in the same manner as in Example 10-3. The concentration of each recombinant protein was measured in the same manner as in Example 10-3, and the mass of purified protein and the mass of purified protein per 1 g of cells were calculated from the volume of the recovered solution and the amount of cells.

The results are illustrated in Fig. 10. The amounts of purified protein per 1 g of cells were 2.10 mg/g for FP120, 0.79 mg/g for FP021, and 3.38 mg/g for FP121-6. The proportion (%) of recombinant protein contained in the extracted protein calculated according to the formula of Example 11-3 was 35% for FP120, 28% for FP021, and 48% for FP121-6.

### <Example 13: Expression of Recombinant Proteins cbFP121-6 and hEGF-FP1-6>

### [13-1. Culture of Transformants]

The transformants E. coli BL21/pETcb121 and E. coli BL21/pEThEGF obtained in Example 9 were each cultured in the same manner as in Example 10-1, sampled before and after IPTG addition as in Example 11-1, and, after completion of the culture step, the cell pellets were collected and stored at -80°C.

### [13-2. Expression Analysis of Recombinant Protein]

The cultured products sampled during the culture step were subjected to electrophoresis using a 15% SDS-PAGE gel in the same manner as in Example 11-2, and the expression status of the recombinant proteins was visualized by Coomassie Blue.

**[Table 11]**

| hEGF-FP1-6, cbFP121 | | | |
|---|---|---|---|
| Lane | Transformant | Condition | Sample amount |
| M | Protein Molecular Weight Marker (Broad) | - | 5µL |
| 1 | E.coli BL21/pEThEG F-1 | Before induction | 10 µL |
| 2 | | After induction | 10 µL |
| 3 4 | E.coli BL21/pETcb1 21 | Before induction | 10 µL |
| | | After induction | 10 µL |

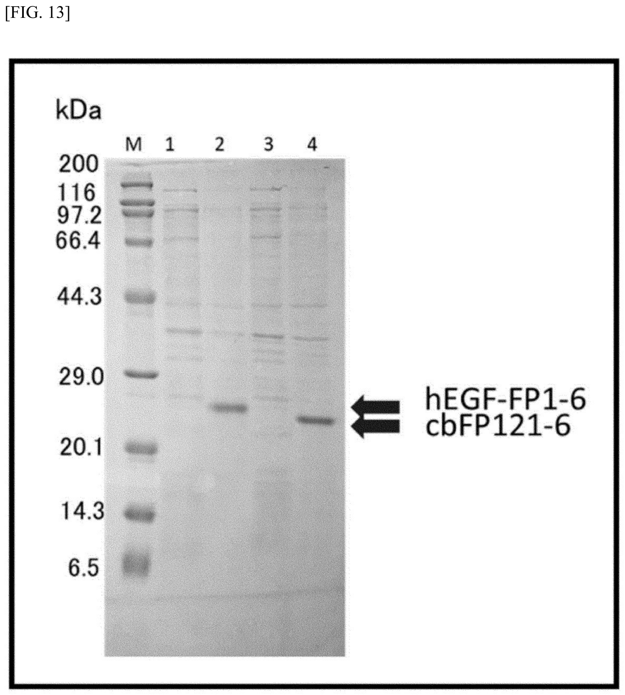

The results are illustrated in Fig. 13. From each of the induced cultures, the expression of each of cbFP121-6 (about 23 kDa) and hEGF-FP1-6 (about 25 kDa) was confirmed.

### [13-3. Preparation of Samples for Extraction and Purification of Recombinant Proteins]

Recombinant proteins were extracted from 0.5 to 0.6 g of the transformants cultured in Example 13-1 in the same manner as in Example 11-2. The extracted cbFP121-6 was clarified using a 0.45 µm filter, neutralized to a pH in a range of 3 to 4 by adding an equal volume of 500 mM sodium acetate, and then used for chromatography purification. The extracted hEGF-FP1-6 was clarified with a 0.45 µm filter, and 9 g of urea was dissolved in 10 mL of the extract, and then used for chromatography purification.

### [13-4. Purification of cbFP121-6]

In order to separate and purify the cbFP121-6 prepared in Example 13-3, cation exchange chromatography was performed in the same manner as described in Example 10-3. However, 2 mL of carrier and, as an equilibration buffer, a 50 mM sodium acetate buffer (pH 5.5) and 250 mM sodium chloride were used, and, after passing the recombinant protein solution prepared in Example 13-3 through the column, the protein was eluted using a 50 mM sodium acetate buffer containing 750 mM sodium chloride. The elution fractions were collected in portions of 1 mL each. For each sample of the elution fractions, A280 measurement and SDS-PAGE analysis were performed to confirm the elution of the recombinant proteins.

**[Table 12]**

| **cbFP121-6** | | | |
|---|---|---|---|
| Lane | Sample | Sample amount | A280 |
| **M** | **Protein Molecular Weight Marker (Broad)** | **5 µL** | - |
| **1** | Extract | **5 µL** | **0.911** |
| **2** | Flow-through fraction | **5 µL** | **0.399** |
| **3** | Wash fraction | **5 µL** | **0.067** |
| **1** | **750mM NaCl** Elution fraction 1 | **5 µL** | **-0.001** |
| **5** | **750mM NaCl** Elution fraction 2 | **5 µL** | **3.810** |
| **6** | **750mM NaCl** Elution fraction 3 | **5 µL** | **3.289** |
| **7** | **750mM NaCl** Elution fraction 4 | **5 µL** | **1.027** |
| **8** | **750mM NaCl** Elution fraction 5 | **5 µL** | **0.265** |

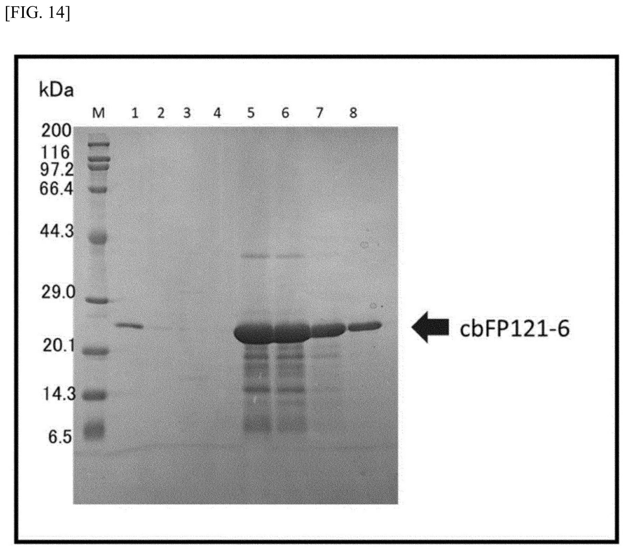

### The results of the SDS-PAGE are illustrated in Fig. 14.

As in Example 10-3, the elution fractions of the recombinant protein were combined, desalted, and the protein concentration was measured. The yield of the purified recombinant protein prepared was about 3.13 mg.

### [13-5. Purification of hEGF-FP1-6]

In order to separate and purify the hEGF-FP1-6 prepared in Example 13-3, cation exchange chromatography was performed in the same manner as described in Example 10-3. However, 2 mL of carrier and an equilibration buffer containing 0.5% acetic acid and 8 M urea were used, and after passing the extract prepared in Example 13-3 through the column, proteins were eluted by sequentially passing 3 CV each of the equilibration buffer (0.5% acetic acid, 8 M urea) containing 50, 100, 150, 200, 250, and 500 mM sodium chloride. The elution fractions were collected in portions of 5 mL each.

For each sample of the elution fractions, A280 measurement and SDS-PAGE analysis were performed to confirm the recombinant proteins.

**[Table 13]**

| **hEGF-FP1-6** | | | |
|---|---|---|---|
| Lane | Sample | Sample amount | A280 |
| **M** | **Protein Molecular Weight Marker (Broad)** | **5 µL** | - |
| **1** | Extract | **5 µL** | **0.4800** |
| **2** | Flow-through fraction | **5 µL** | **0.2940** |
| **3** | Wash fraction | **5 µL** | **0.0435** |
| **4** | **50mM NaCl** Elution fraction | **5 µL** | **0.0105** |
| **5** | **100mM NaC**l Elution fraction | 5 **µL** | **0.0315** |
| **6** | **150mM NaCl** Elution fraction | **5 µL** | **0.0585** |
| **7** | **200mM NaCl** Elution fraction | **5 µL** | **0.0765** |
| **8** | **250mM NaCl** Elution fraction | **5 µL** | **0.1530** |
| **9** | **500mM NaCl** Elution fraction | **5 µL** | **0.0540** |

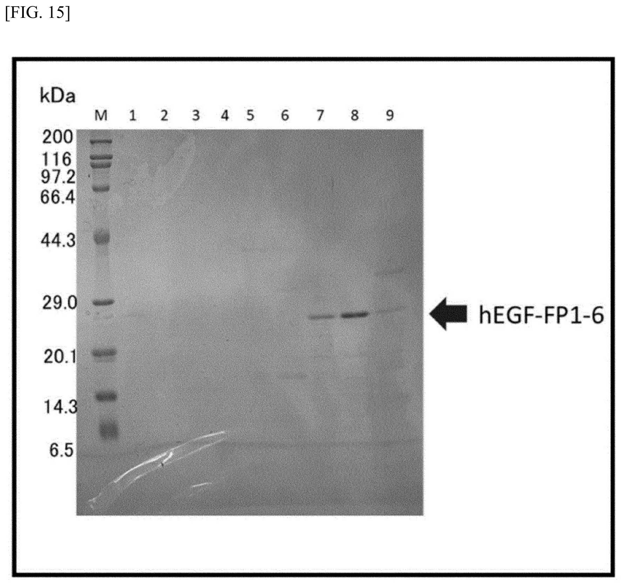

The results of the SDS-PAGE are illustrated in Fig. 15. The recombinant protein was eluted with a buffer containing 200 to 250 mM sodium chloride.

The fraction eluted with a buffer containing 250 mM sodium chloride was dialyzed in the same manner as in Example 10-3, thereby performing desalting and simultaneously removing urea. The protein concentration of the recombinant protein solution was measured in the same manner as in Example 10-3. The yield of the purified recombinant protein prepared was about 568.9 µg.

### <Example 14: DOPA Modification of Tyrosine Residues of FP121-6 (Prototype)>

### [14-1. DOPA Modification of FP121-6 (prototype) by Tyrosinase Treatment]

The conversion of tyrosine residues of the purified FP121-6 (prototype) prepared in Example 10-3 to L-3,4-dihydroxyphenylalanine (DOPA) was performed by incubating at room temperature for 2 to 3 hours in physiological saline (pH 7.4) adjusted to a final composition of 25 mM ascorbic acid, 20 mM borax, 1.0 mg/mL FP121-6 (prototype), and 50 µg/mL tyrosinase (Sigma, USA).

### [14-2. Purification of DOPA-Modified FP121-6 (Prototype)]

In order to separate and purify the DOPA-modified FP121-6, cation exchange chromatography was performed in the same manner as in Example 10-3. However, 2 mL of the carrier and, as the equilibration buffer, a 50 mM sodium acetate buffer (pH 5.5) and 250 mM sodium chloride were used. The reaction solution of Example 14-1 was diluted 10-fold with 50 mM sodium acetate buffer and passed through the equilibrated column, the column was washed, and then the protein was eluted using 50 mM sodium acetate buffer containing 750 mM sodium chloride. For the sample of each elution fraction, the protein was confirmed by measuring A280, and the elution fractions of the protein were combined.

### [14-3. Purification of DOPA-Modified FP121-6 (Prototype)]

In order to confirm the DOPA modification of FP121-6 (prototype), samples of the eluate obtained in Example 14-2 were subjected to electrophoresis using a 15% SDS-PAGE gel, transferred onto a PVDF membrane, and stained with nitro blue tetrazolium (NBT).

**[Table 14]**

| DOPA-modified FP121-6 (prototype) | | |
|---|---|---|
| Lane | Sample | Sample amount |
| M | Protein Ladder One Plus, Triple-color for SDS-PAGE | 5 µL |
| 1 | FP121-6 (prototype) after DOPA modification (before purification) | 7.5 µL |
| 2 | FP1216 (prototype) after DOPA modification (after purification) | 7.5 µL |
| 3 | FP121-6 (prototype) before DOPA modification | 7.5 µL |

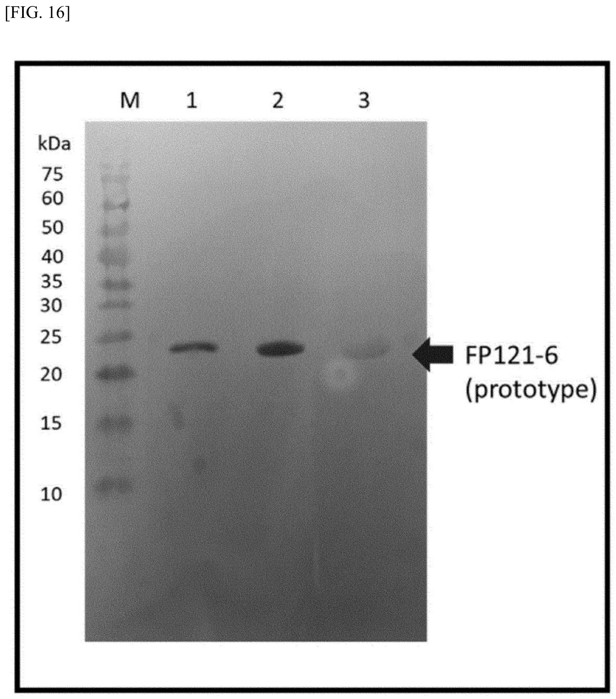

The results of the SDS-PAGE are illustrated in Fig. 16. The purified FP121-6 (prototype) modified with DOPA was visualized as blue-purple bands indicating the formation of NBT formazan (lanes 1 and 2), confirming evidence of the conversion of tyrosine residues to DOPA.

The DOPA-modified FP121-6 (prototype) in which DOPA modification was confirmed was desalted in the same manner as in Example 10-3.

### 3. Evaluation of Performance of Recombinant Protein

### <Example 15: Evaluation of Cell Adhesiveness of FP121-6 (Prototype) and DOPA-Modified Product Thereof>

In order to demonstrate the utility of FP121-6 (prototype) and DOPA-modified FP121-6 (prototype) prepared in Examples 10 and 14 as coating materials for cultured cells, each protein was coated onto a polystyrene (hereinafter, PS) culture plate (WATSON, Japan), and an adhesion assay of the K562 cell line, which is a suspension cell line of chronic myelogenous leukemia, to the plate was performed.

In addition, for comparison, an adhesion assay was also performed for FP151, which is a known adhesive substance. Note that FP151 (SEQ ID NO: 73) was expressed using a pET22b(+) vector in which the FP151 gene (SEQ ID NO: 74) was inserted, together with the same cells (BL21(DE3)) as in Example 9, and the expressed product was purified by metal affinity chromatography using Ni Sepharosetm 6 Fast Flow (Cytiva).

The recombinant protein was dissolved under acidic conditions in a 0.5% (v/v) acetic acid aqueous solution to prevent adsorption during storage. Before being adsorbed onto a 96-well PS plate, the protein was neutralized with 0.1 M sodium bicarbonate aqueous solution (pH 8.2) in accordance with the method recommended in the instruction manual of the commercially available cell and tissue adhesive "Cell-Tak" (trade name, Coming, USA). Each well (well area: 0.32cm²) was coated by adding each recombinant protein at 3.5 µg/cm² and allowing it to stand overnight at room temperature. The wells were washed twice with sterile water and air-dried in a clean bench. As a positive control, "Cell-Tak" was used, and as a negative control, the same volume of 0.5% (v/v) acetic acid aqueous solution was added, and the wells were treated in the same manner.

K562 cells were seeded into each well at 2 × 10⁴ cells per well and cultured for 24 hours in RPMI1640 medium (NACALAI TESQUE, INC., Japan) containing 10% (v/v) fetal bovine serum (FBS; BioWest, France) under 5% CO₂ at 37°C. After removing the medium, the wells were washed once with fresh medium to remove non-adherent cells, the adherent cells were subjected to color development using the "Cell Counting Kit-8" (Dojindo Laboratories Co., Ltd., Japan), and then, the absorbance (A450) at 450 nm was measured. The cell adhesion rate was calculated by the calculation formula "cell adhesion rate (%) = (absorbance of adherent cells only - background absorbance)/(absorbance before washing - background absorbance) × 100".

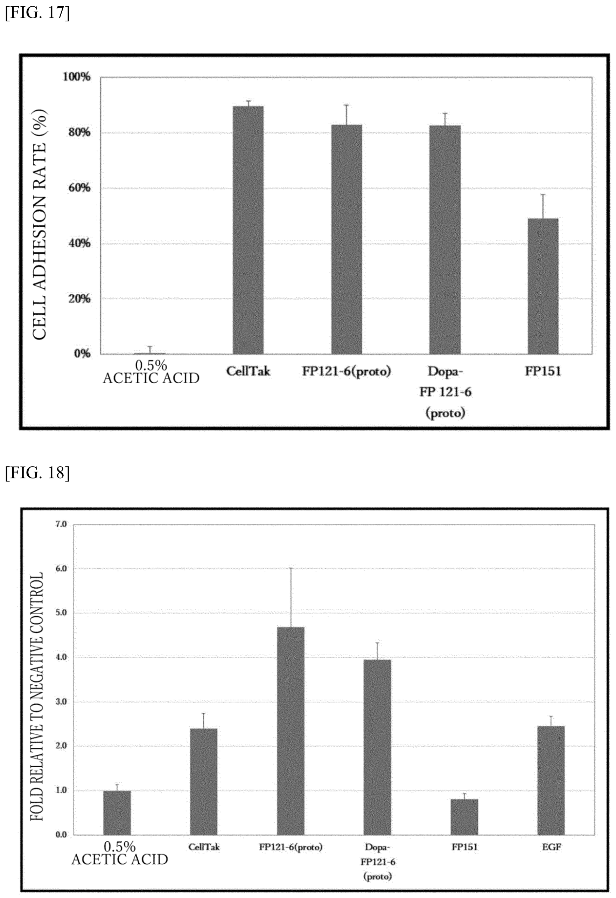

The results are illustrated in Fig. 17. The adhesion rates to cells were 83 ± 7% for FP121-6 (prototype) and 83 ± 4% for DOPA-modified FP121-6 (prototype), showing cell adhesion rates comparable to that of the positive control Cell-Tak (90 ± 2%). In addition, both FP121-6 (prototype) and DOPA-modified FP121-6 (prototype) exhibited about 80% higher cell adhesion rates compared with the negative control (0.5 ± 2%), indicating significantly higher cell adhesion performance. Furthermore, FP121-6 (prototype) and DOPA-modified FP121-6 (prototype) exhibited strong cell adhesion performance, which was more than about 30% higher than that of FP151.

### <Example 16: Evaluation of Cell Proliferation-Stimulating Effect of FP121-6 (Prototype) and DOPA-Modified Product Thereof>

The cell proliferation-stimulating effects of FP121-6 (prototype) and DOPA-modified FP121-6 (prototype) prepared in Examples 10 and 14 were evaluated based on the proliferation of HeLa cells, a human cervical cancer cell line, in the presence of each protein. In addition, for comparison, FP151 was also used in the present experiment.

2 × 10⁶ cells were seeded in a 10 cm dish and cultured overnight at 37 °C in the presence of 5% CO₂ in Dulbecco's Modified Eagle's Medium (hereinafter, DMEM medium; NACALAI TESQUE, INC., Japan) containing 10% (v/v) fetal bovine serum (FBS), and then, the medium was replaced with serum-free DMEM medium, and the cells were further cultured overnight. 5 × 10³ cells adapted to serum-free medium were seeded into each well of a 96-well plate (Nippon Genetics, Japan) and cultured for 3 days at 37°C in the presence of 5% CO₂ in DMEM medium containing each recombinant protein or Cell-Tak at a final concentration of 11.2 µg/well. As a positive control, "human recombinant epidermal growth factor protein" (Oriental Yeast Co., Ltd., Japan) was added to the medium at 40 ng/mL, and as a negative control, the same volume of 0.5% (v/v) acetic acid aqueous solution was added, followed by culturing in the same manner.

After removing the culture supernatant and washing with DMEM medium once, A450 was measured in the same manner as in Example 15 using "Cell Counting Kit-8". The cell proliferation-stimulating effect was calculated as (absorbance for each condition - background absorbance)/(absorbance for negative control - background absorbance).

The results are illustrated in Fig. 18. The cell proliferation-stimulating effect was 4.7 ± 1.3-fold for FP121-6 (prototype) and 3.9 ± 0.4-fold for DOPA-modified FP121-6 (prototype) relative to the negative control, and both showed high cell proliferation-stimulating effects. In addition, FP151, which is a known substance, exhibited no cell proliferation-stimulating effect, and both FP121-6 (prototype) and DOPA-modified FP121-6 (prototype) had approximately 1.7- to 2-fold higher cell proliferation-stimulating effects than Cell-Tak, which is a known product.

In addition, from the above, it was found for the first time that FP2 known as an adhesive protein has a cell proliferation-stimulating ability.

### <Example 17: Comparison of Cell Adhesion Rates According to Difference in Number of Repeats of FP1 Sequence in FP121>

For the recombinant proteins FP121-3, FP121-6, and FP121-9, which differ in the number of repeats of the FP1 sequence at both ends of FP2 and were prepared in Example 11, a cell adhesion assay was performed in the same manner as in Example 15. FP121-6 (prototype) was used as a comparative control. A 0.5% (v/v) acetic acid aqueous solution of the same volume was used as a negative control.

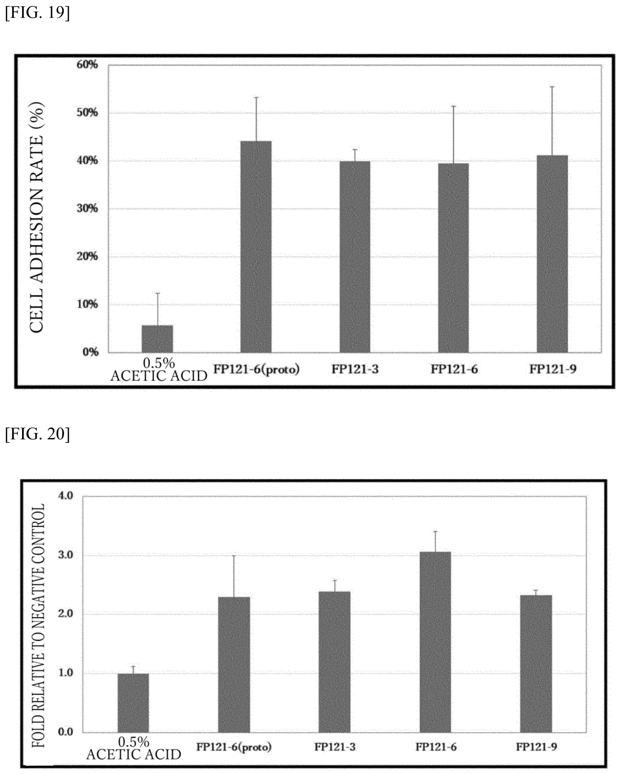

The results are illustrated in Fig. 19. The cell adhesion rate was 40 ± 3% for FP121-3, 39 ± 12% for FP121-6, 41 ± 14% for FP121-9, and 44 ± 9% for FP121-6 (prototype), and all showed almost the same adhesion rates regardless of the number of repeats. FP121-6 without a His tag exhibited a cell adhesion rate comparable to that of FP121-6 (prototype). From the above, it was shown that the number of repeats of the FP1 sequence does not affect the cell adhesion rate, and that the presence or absence of a His tag has little effect on the cell adhesion rate.

### <Example 18: Evaluation of Cell Proliferation-Stimulating Effects According to Difference in Number of Repeats of FP1 Sequence in FP121>

A cell proliferation assay using the HeLa cell line was performed for FP121-3, FP121-6, and FP121-9 used in Example 17, in the same manner as in Example 16. FP121-6 (prototype) was used as a comparative control. A 0.5% (v/v) acetic acid aqueous solution of the same volume was used as a negative control.

The results are illustrated in Fig. 20. The cell proliferation-stimulating effects were 2.4 ± 0.2-fold for FP121-3, 3.1 ± 0.3-fold for FP121-6, 2.3 ± 0.1-fold for FP121-9, and 2.3 ± 0.7-fold for FP121-6 (prototype) relative to the negative control. From the above, it was shown that each of FP121-3, FP121-6, FP121-6 (prototype), and FP121-9 had high cell proliferation-stimulating activity. In addition, it was also shown that, in particular, FP121-6 had higher cell proliferation-stimulating effect than those having other numbers of repeats.

### <Example 19: Comparison of Cell Adhesion Rates Between FP1 Fusion Protein at Both Termini and FP1 Fusion Protein at One Terminus>

In order to evaluate differences in cell adhesion performance depending on whether the FP1 sequence was fused to FP2 at only the N-terminus (FP120), only the C-terminus (FP021), or at both termini, a cell adhesion assay was performed in the same manner as in Example 15. As recombinant proteins in which FP1 was fused to both termini, FP121-6 and FP121-3 prepared in Example 11 were used. In addition, FP120 and FP021 prepared in Example 12 were used. A 0.5% (v/v) acetic acid aqueous solution of the same volume was used as a negative control.

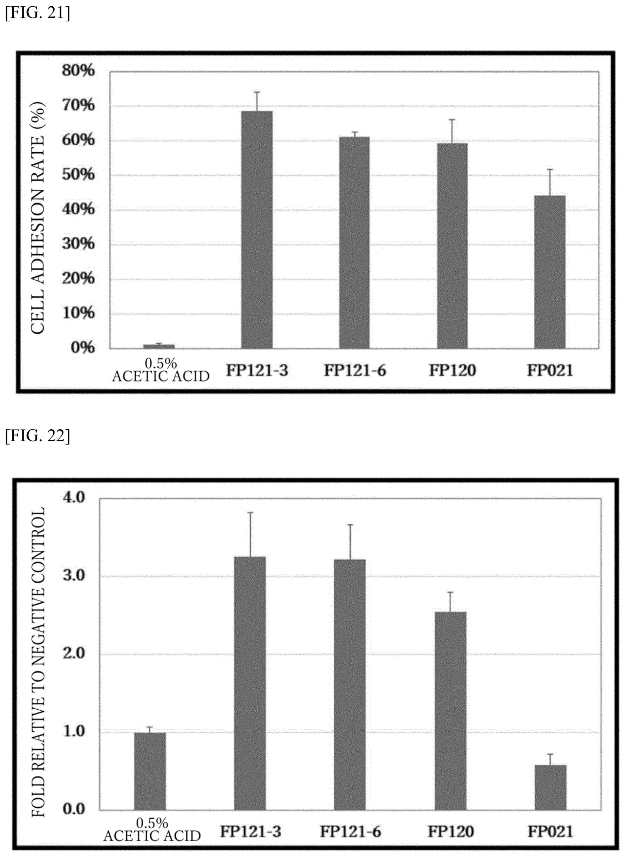

The results are illustrated in Fig. 21. The cell adhesion rates were 69 ± 5% for FP121-3, 61 ± 2% for FP121-6, and 59 ± 7% for FP120, and these exhibited equivalent adhesion rates, whereas FP021 showed a cell adhesion rate of 44 ± 8%, which was lower by about 15% (p < 0.05). From these results, it was shown that proteins in which FP1 was fused to both termini had higher cell adhesion performance than proteins in which the FP1 sequence was fused only to the C-terminus. In addition, it was considered that fusing an adhesive molecule to the C-terminus of a functional molecule such as FP2 reduces the adhesiveness of the complex of the functional molecule and the adhesive molecule; however, surprisingly, it was shown that fusing adhesive molecules to both the N-terminus and the C-terminus of the functional molecule maintained or enhanced the adhesiveness of the complex molecule.

### <Example 20: Comparison of Cell Proliferation-Stimulating Effect Between FP1 Fusion Protein at Both Termini and FP1 Fusion Protein at One Terminus>

In order to evaluate differences in cell proliferation-stimulating effect depending on whether the FPl sequence was fused to FP2 at only the N-terminus (FP120), only the C-terminus (FP021), or at both termini, a cell proliferation assay using HeLa cells was performed in the same manner as in Example 16. FP121-3 and FP121-6 prepared in Example 11 were used. FP120 and FP021 prepared in Example 12 were used. A 0.5% (v/v) acetic acid aqueous solution of the same volume was used as a negative control in the same manner as in Example 17.

The results are illustrated in Fig. 22. The cell proliferation-stimulating effect was 3.3 ± 0.6-fold for FP121-3 and 3.2 ± 0.4-fold relative to the negative control, and a high proliferation-stimulating effect was exhibited in both cases. On the other hand, FP120 as a single-end fusion protein showed 2.6 ± 0.3-fold, and FP021 as a single-end fusion protein showed 0.6 ± 0.1-fold, and both exhibited lower proliferation-stimulating effect compared with that of the fusion proteins at both ends. From these results, it was shown that, in order to more effectively enhance the cell proliferation-stimulating effect of FP2, fusing an adhesive molecule to both the N-terminus and the C-terminus of a functional molecule such as FP2 (the case of both ends) is superior to fusing an adhesive molecule to only the C-terminus or only the N-terminus of the functional molecule (the case of single end). In addition, it was considered that fusing an adhesive molecule to the C-terminus of a functional molecule such as FP2 lowers the cell proliferation-stimulating activity of the complex of the functional molecule and the adhesive molecule; however, surprisingly, it was shown that fusing adhesive molecules to both the N-terminus and the C-terminus of the functional molecule enhanced the cell proliferation-stimulating activity of the complex molecule compared with the case of a single end.

### <Example 21: Evaluation of Cell Adhesiveness of hEGF-FP1-6>

A cell adhesion assay was performed for the hEGF-FP1-6 protein prepared in Example 13 in the same manner as in Example 15. A 0.5% (v/v) acetic acid aqueous solution of the same volume was used as a negative control. Note that hEGF-FP1-6 is a recombinant protein having human epidermal growth factor (hEGF) as a functional peptide portion and the FP1 sequence as an adhesive peptide portion.

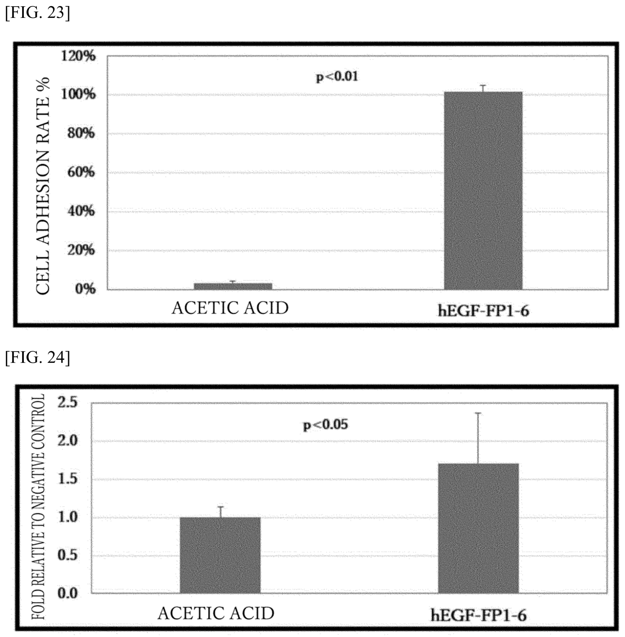

The results are illustrated in Fig. 23. The cell adhesion rate was 3.3 ± 1% for the negative control, whereas the hEGF-FP1-6 group showed 102 ± 3%, exhibiting a significantly higher cell adhesion rate (p < 0.01). From these results, it was shown that hEGF-FP1-6 has high cell adhesion performance by being flanked with FP1.

### <Example 22: Evaluation of Cell Proliferation-Stimulating Effect of hEGF-FP1-6 on HeLa Cells>

A cell proliferation assay using a HeLa cell line was performed for the hEGF-FP1-6 protein prepared in Example 13, in the same manner as in Example 16, in order to verify the retention of the function of the functional molecule. A 0.5% (v/v) acetic acid aqueous solution of the same volume was used as a negative control.

The results are illustrated in Fig. 24. The cell proliferation-stimulating effect of the hEGF-FP1-6 group was 1.7 ± 0.7-fold that of the negative control, showing significantly higher cell proliferation (p < 0.05). From these results, it was shown that the high cell proliferation-stimulating effect of rhEGF was also retained by hEGF-FP1-6, and that, in the recombinant protein of the present embodiment, the functional peptide portion retained its original function without loss of function due to the adhesive peptide portion, even when adhesive peptide portions such as the FP1 sequence were fused to both ends.

### <Example 23: Verification of Cell Adhesion Performance After Long-Term Storage of Plastic Agents Coated with FP121-6 and the Like>

The presence or absence of cell adhesion performance after long-term storage was verified for plastic materials coated with FP121-3, FP121-6, FP121-9, FP151, and DOPA-modified FP121-6 (prototype) prepared in Examples 11, 14, and 15. Into non-plasma-treated PS 96-well plates, Cell-Tak, FP121-3, FP121-6, FP121-9, FP151, DOPA-modified FP121-6 (prototype), the same volume of 0.5% acetic acid aqueous solution as a negative control group, and BSA, each diluted with a 0.1 M sodium bicarbonate aqueous solution, were added to 3 wells each so as to provide 3.5 µg/cm², and allowed to stand at 37°C for 2 hours. After washing twice with sterile water, the plates were air-dried for 30 minutes in a clean bench, and then stored at 25°C under light-shielded conditions. The storage period was 6 months and 3 months. After the storage period, a cell adhesion assay of the suspension K562 cell line to the plates was performed in the same manner as in Example 17.

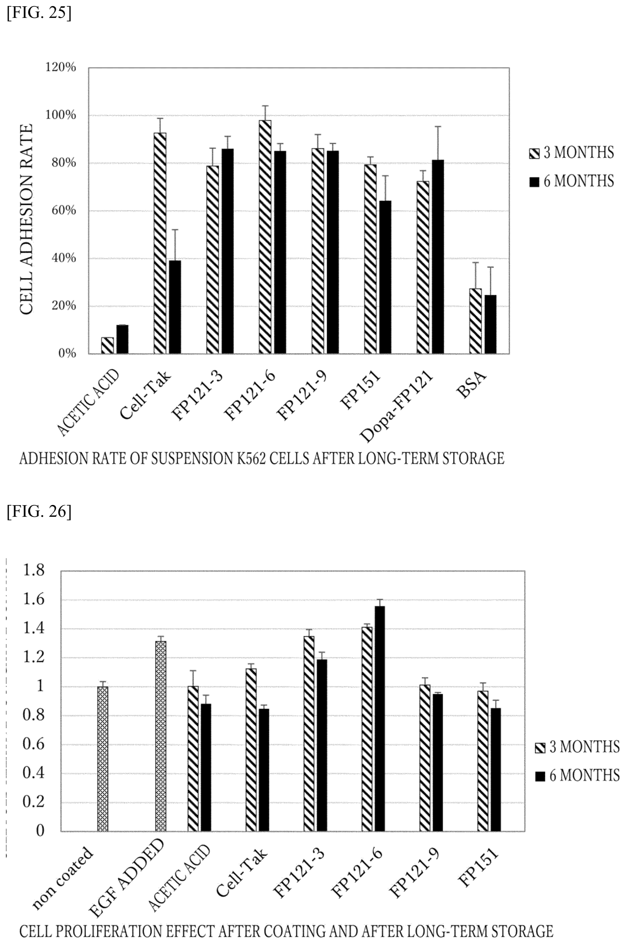

The results are illustrated in Fig. 25. In the plates coated with Cell-Tak, the cell adhesion rate after 3 months of storage was 93 ± 7% (vs. BSA, p < 0.01), whereas after 6 months of storage it decreased to 39 ± 5%, less than half of the original cell adhesion performance (p = 0.33). On the other hand, in the plates coated with FP121-6, the cell adhesion rate was 98 ± 6% after 3 months of storage and 85 ± 3% after 6 months of storage, indicating that the adhesion performance was maintained (both p < 0.01). In addition, FP121-3, FP121-9, and DOPA-modified FP121-6 (prototype) also maintained 80% adhesion performance even after 6 months of storage. FP151 showed a slight decrease in adhesion performance. From these results, it was shown that plastic materials coated with FP121-3, FP121-6, FP121-9, or DOPA-modified FP121-6 can be stored at room temperature for up to 6 months, and that the cell adhesion performance was maintained even after long-term storage.

### <Example 24: Verification of Cell Proliferation Effect After Long-Term Storage of Plastic Agents Coated with FP121-6 and the Like>

The presence or absence of cell proliferation effect after long-term storage was verified for plastic materials coated with FP121-3, FP121-6, FP121-9, FP151, and DOPA-modified FP121-6 (prototype) prepared in Examples 11, 14, and 15. Into non-plasma-treated PS 96-well plates, Cell-Tak, FP121-3, FP121-6, FP121-9, FP151, and same volume of 0.5% acetic acid aqueous solution as a negative control group, each diluted with a 0.1 M sodium bicarbonate aqueous solution, were added to 3 wells each so as to provide 3.5 µg/cm², and allowed to stand at 37°C for 2 hours. After washing twice with sterile water, the plates were air-dried for 30 minutes in a clean bench, and then stored at 25°C under light-shielded conditions. The storage period was 6 months and 3 months. After the storage period, a cell proliferation assay using a HeLa cell line was performed in the same manner as in Example 18. As a negative control, the same volume of a 0.5% (v/v) acetic acid aqueous solution was coated in the same manner as described above. EGF was added to the medium as a positive control.

The results are illustrated in Fig. 26. Relative to the cell number in non-coated wells, the addition of EGF caused the HeLa cells to proliferate 1.3-fold, indicating that they responded to EGF. After 3 months of storage for each coating agent, no proliferation was observed with Cell-Tak, which showed 1.1 ± 0.0-fold; however, in the conditions coated with FP121-3 or FP121-6, cell proliferation effects were observed at 1.3 ± 0.0-fold and 1.4 ± 0.0-fold, respectively (both vs. the negative control, p < 0.01). No proliferation effect was observed with FP121-9 or FP151. After 6 months of storage for each coating agent, Cell-Tak showed 0.85 ± 0.0-fold, indicating that the cell number had decreased instead. In the condition coated with FP121-3, the value was 1.2 ± 0.0-fold, indicating that the cell proliferation effect had decreased compared with that after 3 months of storage. In the condition coated with FP121-6, the value was 1.6 ± 0.0-fold, showing a cell proliferation effect, which was further enhanced compared with that after 3 months of storage (p < 0.001). In the condition coated with FP121-9 or FP151, the number of cells had slightly decreased. From these results, it was shown that, in plastic materials coated with FP121-3 or FP121-6, the cell proliferation effect was maintained even during storage at room temperature for 6 months, and that, in particular, in plastic materials coated with FP121-6, the cell proliferation effect was not only maintained but also enhanced even during storage at room temperature for 6 months, indicating that each is excellent for long-term storage as a cell coating agent.

### <Example 25: Gene Sequence for Recombinant Production of hNGF-FP1-6, Vector Production, and Production of Transformants Producing Fusion Protein>

A fusion protein (SEQ ID NO: 75) in which the FP1 motif was repeated 6 times at both ends of human-derived hNGF was designed. In order to mass-produce this fusion protein using a T7 promoter, the hNGF-FP1-6 gene (SEQ ID NO: 76) was inserted between the Ndel and XhoI restriction enzyme sites of the pET22b(+) vector (Novagen, USA), thereby producing a pEThNGF-1 vector. Transformants producing a fusion protein were produced in the same manner as in Example 9 to obtain E. coli BL21/pEThNGF-1.

### <Example 26: Gene Sequence and Vector Production for Recombinant Production of hbFGF-FP1-6>

A fusion protein (SEQ ID NO: 77) in which the FP1 motif was repeated 6 times at both ends of human-derived hbFGF was designed. In order to mass-produce this fusion protein using a T7 promoter, the hbFGF-FP1-6 gene (SEQ ID NO: 78) was inserted between the NdeI and XhoI restriction enzyme sites of the pET22b(+) vector (Novagen, USA), thereby producing a pEThbFGF-1 vector. Transformants producing a fusion protein were produced in the same manner as in Example 9 to obtain E. coli BL21/pEThbFGF-1.

### <Example 27: Expression of Recombinant Proteins hNGF-FP1-6 and hbFGF-FP1-6>

### [27-1. Culture of Transformants]

After culturing the transformants (E. coli BL21p/EThNGF-1 and E. coli BL21/pEThbFGF-1) in 2×YT medium, when the OD600 of the culture medium reached around 1.5, IPTG was added at a final concentration of 1 mM to induce the expression of the recombinant adhesive proteins. The culture conditions were so that 1 mL of a preculture medium, obtained by culturing in 5 mL of 2×YT medium (containing 100 µg/mL ampicillin) in a sterilized 50-mL tube for 5 to 16 hours, was inoculated into a 500-mL baffled flask containing 100 mL of LB medium for the main culture. The culturing temperature was set at 37°C. After completion of the culture step, the culture medium was centrifuged at 3,000 × g for 15 minutes to collect the cell pellet, which was stored at -80°C until use in the next experiment.

### [27-2. Expression Analysis of Recombinant Protein]

The culture separated during the culture step was adjusted to an E coli concentration of OD600 ≈ 1.0 (±0.1), and 50 µL of the bacterial suspension was centrifuged at 15,000 rpm for 5 minutes at 4°C to pellet the cells. To this cell pellet, 50 µL of an electrophoresis sample buffer containing urea (0.0625 M Tris-HCl, 2% (w/v) SDS, 10% (v/v) glycerol, 0.005% (w/v) BPB, 5% (v/v) 2-ME, and 8 M urea) was added and suspended to prepare an E. coli lysate. The E. coli lysate was subjected to electrophoresis using a 15% SDS-PAGE gel, and the expression state of the recombinant protein was visualized with Coomassie Blue. From the results of SDS-PAGE analysis, the expression of each of hNGF-FP1-6 (about 30 kDa) and hbFGF-FP1-6 (about 35 kDa) was confirmed from the induced cultures.

### [27-3. Preparation of Samples for Extraction and Purification of Recombinant Proteins]

The recombinant protein was extracted by adding 10 mL of a buffer containing 0.5% acetic acid, 8 M urea, and 150 mM NaCl to 1 g of the cells collected in Example 27-1. The insoluble fraction in the extract was removed by centrifugation at 16,000 × g and 4°C for 15 minutes, and then clarification with a 0.45 µm filter. In order to separate and purify the recombinant protein, cation exchange chromatography was performed. As a chromatography carrier, CM Sepharose (registered trademark) Fast Flow (Cytiva) was used. The column was filled with 2 mL of the carrier, and then equilibrated by passing an equilibration buffer (0.5% acetic acid, 8 M urea, 150 mM NaCl). The extract of recombinant protein (after clarification) was passed through the column, and then the equilibration buffer was passed twice by 5CV to wash the column. The recombinant protein was eluted by passing 3CV of an elution buffer (0.5% acetic acid, 8 M urea, and 250 mM NaCl). The elution fractions were each collected in an amount of 1 mL. Each sample during the purification step was analyzed by measuring absorbance at an absorption wavelength of 280 nm and by SDS-PAGE to confirm the elution of the recombinant protein. The fractions eluted with a buffer containing 250 mM sodium chloride were dialyzed at room temperature against a 0.5% acetic acid solution (Spectra/Por 1, REPLIGEN, USA) to perform desalting and urea removal. The solution after dialysis was pulverized by freeze-drying, and then dissolved and adjusted in 0.5% acetic acid to a protein concentration of 1 mg/ml.

### <Example 28: Verification that Growth Factor Fusion Proteins Flanked by FP1 Sequences at Both Ends Have Cell Adhesiveness>

In order to demonstrate the usefulness of hNGF-FP1-6 purified in Example 27 as a cell culture coating material, it was coated onto PS culture plates, and a cell adhesion assay using the suspension K562 cell line was performed on the plates.

A 1 mg/mL hNGF-FP1-6 protein solution diluted with a 0.5% acetic acid aqueous solution was added to each well so that the amount per well was 2 µg, and, as comparative controls, FP121-6, FP151, and Cell-Tak were added to wells in the same manner. As a negative control experiment, only a 0.5% acetic acid aqueous solution was used. The 96-well plates to which these solutions were added were dried at 37°C for 2 days or longer to completely evaporate the contained acetic acid and moisture. The K562 cell line cultured in RPMI1624 medium containing 10% FBS was seeded at 20,000 cells per well and cultured in a 5% CO₂ incubator for 24 hours. After removing the medium, the wells were washed once with fresh medium to remove non-adherent cells, the adherent cells were subjected to color development using the "Cell Counting Kit-8" (Dojindo Laboratories Co., Ltd., Japan), and then, the absorbance (A450) at 450 nm was measured. The cell adhesion rate was calculated by the calculation formula "cell adhesion rate (%) = (absorbance of adherent cells only - background absorbance)/(absorbance before washing - background absorbance) × 100".

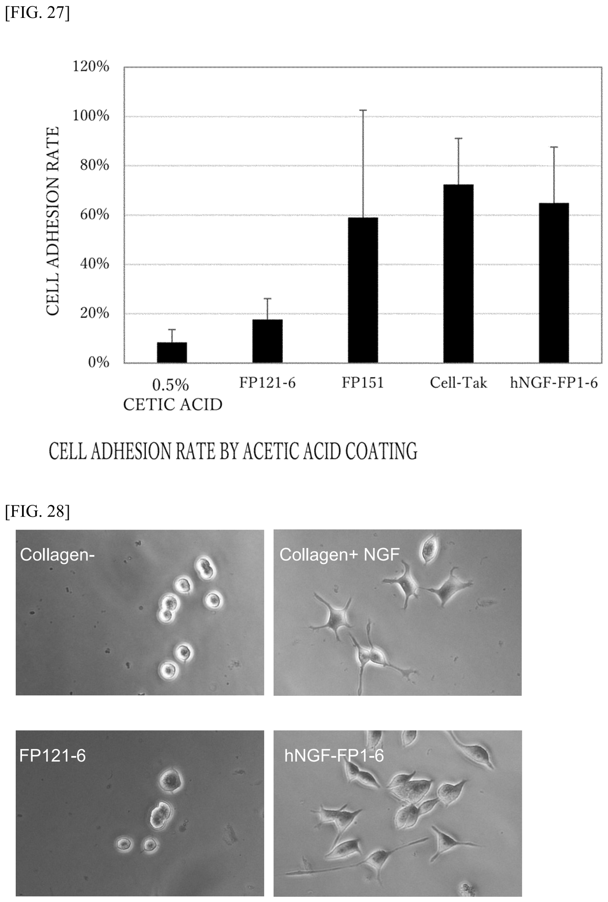

The results are illustrated in Fig. 27. When fusion proteins suspended in acetic acid were coated, the cell adhesion rates were 18 ± 8.4% for FP121-6, which was low, 59 ± 44% for FP151, 73 ± 19% for Cell-Tak (vs. negative control group, p < 0.01), and 65 ± 23% for hNGF-FP1-6 (p < 0.05), showing cell adhesiveness comparable to that of Cell-Tak. From these results, it was shown that hNGF-FP1-6 can exhibit cell adhesiveness when suspended and coated in a 0.5% acetic acid aqueous solution.

### <Example 29: Verification that Growth Factor Fusion Proteins Flanked by FP1 Sequences at Both Ends Have Corresponding Growth Factor Effect>

For hNGF-FP1-6 purified in Example 27, whether the fusion protein has the same effect as nerve growth factor (NGF) was verified using the rat adrenal pheochromocytoma-derived PC12 cell line P3 (hereinafter, the PC12 cell line). The PC12 cell line is widely used in neuronal differentiation induction assays with NGF (Proc. Natl. Acad. Sci. U.S.A. 1976 Jul; 73 (7): 2424-8).

Cells coated with hNGF-FP1-6 or FP121-6 by the method performed in Example 28 exhibited morphological changes to stellate cells and neurite outgrowth (Fig. 28, lower right). The same procedure was performed for FP151, but the cells remained in a spherical morphology. From these results, it was shown that hNGF-FP1-6 had the ability to induce neuronal differentiation as NGF when used as a cell coating agent. It was shown that, by flanking a growth factor protein at both ends with FP1 sequences, the corresponding growth factor can be made to adhere to a material and the growth factor effect can be retained.

## Claims

1. A recombinant protein comprising: a functional peptide portion having growth factor activity; and an adhesive peptide portion fused to an N-terminal side and an adhesive peptide portion fused to a C-terminal side of the functional peptide portion.

2. The recombinant protein according to claim 1, wherein the functional peptide portion is a functional peptide derived from a human.

3. The recombinant protein according to claim 1, wherein the adhesive peptide portion on the N-terminal side or the C-terminal side is an adhesive peptide derived from a mussel.

4. The recombinant protein according to claim 1, wherein two or more of the adhesive peptide portions are present on each of the N-terminal side and the C-terminal side of the functional peptide portion.

5. The recombinant protein according to claim 1, wherein the functional peptide portion has cell proliferation-stimulating activity.

6. The recombinant protein according to claim 1, wherein the functional peptide portion has an amino acid sequence set forth in any one of SEQ ID NOs: 1 to 12, 79, or 80.

7. The recombinant protein according to claim 1, wherein the adhesive peptide portion has an amino acid sequence set forth in any one of SEQ ID NOs: 13 to 23.

8. A nucleic acid encoding the recombinant protein according to any one of claims 1 to 7.

9. An expression vector comprising the nucleic acid according to claim 8 in a configuration capable of expression.

10. A transformant transformed with the expression vector according to claim 9.

11. A method for producing the recombinant protein, the method comprising culturing a transformant comprising the expression vector according to claim 9 and recovering the recombinant protein from the culture.

12. A composition comprising the recombinant protein according to any one of claims 1 to 7.

13. The composition according to claim 12, wherein the composition is a medical adhesive.

14. The composition according to claim 12, wherein the composition is a coating agent for cell culture.

15. The composition according to claim 12, wherein the composition is a cell proliferation-stimulating agent.
